# EUROPEAN PATENT SPECIFICATION

(11) **EP 1 115 828 B1**
(45) Date of publication and mention of the grant of the patent: **12.04.2006**
(21) Application number: 99923145.9
(22) Date of filing: 17.05.1999
(51) Int. Cl.: C11D 3/386, C12N 9/42, C11D 3/37

(54) **LAUNDRY DETERGENT AND/OR FABRIC CARE COMPOSITIONS COMPRISING A CHEMICAL ENTITY WHICH CONTAINS A DEPOSITION AID WITH A HIGH AFFINITY FOR CELLULOSE, A SURFACTANT AND A PROTEASE**
WASCHMITTEL UND/ODER GEWEBEBEHANDLUNGSZUSAMMENSETZUNGEN ENTHALTEND EINE CHEMISCHE EINHEIT, DIE EIN ABSETZUNGSHILFSMITTEL MIT HOHER CELLULOSEAFFINITÄT ENTHÄLT, SOWIE EIN TENSID UND PROTEASE
COMPOSITIONS DE DETERGENTS A LESSIVE ET/OU DE SOIN DES TISSUS, COMPRENANT UNE ENTITE CHIMIQUE QUI CONTIENT UN ADJUVANT DE DEPOT AYANT UNE FORTE AFFINITE POUR LA CELLULOSE, UN TENSIOACTIF ET UNE PROTEASE

(30) Priority: 30.09.1998 WO PCT/US98/20491
(43) Date of publication of application: 18.07.2001
(73) Proprietor: THE PROCTER & GAMBLE COMPANY, Cincinnati, Ohio 45202 (US)
(72) Inventor: BAECK, Andre, Cesar, B-2820 Bonheiden (BE); SMETS, Johan, B-3210 Lubbeek (BE); BUSCH, Alfred, B-1840 Londerzeel (BE); BOYER, Stanton, Lane, Fairfield, OH 45014 (US)
(74) Representative: Morelle, Evelyne Charlotte Isabelle
(86) International application number: PCT/US1999/010853
(87) International publication number: WO 2000/018864

(56) References cited:
- EP-A- 0 747 471
- EP-A- 0 755 999
- WO-A-95/12655
- WO-A-97/24427
- WO-A-98/00500

## Description

### Field of the Invention

The present invention relates to laundry detergent and/or fabric care compositions comprising a chemical entity, a surfactant and a protease; wherein the chemical entity comprises a deposition aid having a high affinity for cellulose and a benefit agent.

### Background of the invention

Modern laundry detergent and/or fabric care compositions comprise common detergent ingredients such as anionic, nonionic, cationic, amypholytic, zwitterionic, and/or semi-polar surfactants; as well as enzymes such proteases, cellulases, lipases, amylases, and/or peroxidases. Such laundry detergent and/or fabric care compositions may further comprise various detergent ingredients having one or more purposes in obtaining fabrics which are not only clean, fresh and sanitised but also have retained appearance and integrity. Therefore, benefit agents such as perfumes, hygiene agents, insect control agents, bleaching agents, fabric softeners, dye fixatives, soil release agents, and fabric brightening agents have been incorporated into laundry detergent and/or fabric care compositions. In using such detergent components, it is important that some of these compounds deposit on the fabrics so as to be effective during or after the laundering and/or fabric care process.
However, there always remains a need to formulate laundry detergent and/or fabric care compositions providing improved cleaning performance.

The above objective has been met by formulating a laundry detergent and/or fabric care composition comprising a chemical entity, a surfactant and a protease; wherein the chemical entity comprises a deposition aid having a high affinity for cellulose and a benefit agent.

WO 91/10732 describes novel derivatives of cellulase enzymes combining a core region derived from an endoglucanase producible by a strain of *Bacillus* spp., NICMB 40250 with a Cellulose Binding Domain (CBD) derived from another cellulase enzyme, or combining a core region derived from another cellulase enzyme with a CBD derived from said endoglucanase, for improved binding properties. WO94/07998 describes cellulase variants of a cellulase classified in family 45, comprising a CBD, a Catalytically Active Domain (CAD) and a region linking the CBD to the CAD, wherein one or more amino acid residues have been added, deleted or substituted and /or another CBD is added at the opposite end of the CAD. WO95/16782 relates to the cloning and high level expression of novel truncated cellulase proteins or derivatives thereof in *Trichoderma longibrachiatum* comprising different core regions with several CBDs. WO97/01629 describes cellulolytic enzyme preparation wherein the mobility of the cellulase component may be reduced by adsorption to an insoluble or soluble carrier e.g. via the existing or newly introduced CBD. WO97/28243 describes a process for removal or bleaching or soiling or stains from cellulosic fabrics wherein the fabric is contacted in aqueous medium with a modified enzyme which comprises a catalytically active amino acid sequence of a non-cellulolytic enzyme selected from amylases, proteases, lipases, pectinases and oxidoreductases, linked to an amino acid sequence comprising a cellulose binding domain and a detergent composition comprising such modified enzyme and a surfactant. WO98/00500 discloses a composition comprising a protein deposition aid having a high affinity for fibers or a surface and having a benefit agent attached / adsorbed thereto.

Recent publications on protease enzymes encompass : WO98/13461 describes liquid laundry detergent compositions comprising detersive surfactants, active proteolytic enzyme, and a proteolytic inhibitor; WO98/54285 discloses laundry detergent bar compositions comprising soap, anionic synthetic surfactants, proteases, stabilizing agent, builder and detersive ingredients; WO99/04016 discloses cleaning compositions comprising mutated cysteine proteases with reduced proteolytic activity; EP 755 999 is directed to detergent compositions comprising an oxidative stability-enhanced amylase and a specific protease; EP 839 905 describes foam compositions comprising a surfactant system and a protease enzyme.

However, none of these documents disclose a laundry detergent and/or fabric care composition for improved cleaning performance, comprising a chemical entity, a surfactant and a protease; wherein the chemical entity comprises a deposition aid having a high affinity for cellulose and a benefit agent.

### Summary of the invention

The present invention relates to laundry detergent and/or fabric care compositions for improved cleaning and/or fabric care performance, comprising a chemical entity, a surfactant and a protease; wherein the chemical entity comprises an amino acid sequence comprising a cellulose binding domain, and a benefit agent.

### Detailed description of the invention

The present invention relates to laundry detergent and/or fabric care compositions comprising a chemical entity, a surfactant and a protease; wherein the chemical entity comprises a deposition aid with a high affinity for cellulose and a benefit agent.

It has now surprisingly being found that laundry detergent and/or fabric care compositions comprising the combination of a chemical entity with a surfactant and a protease, demonstrates improved fabric care performance including anti-wrinkling, anti-bobbling and anti-shrinkage properties, as well as fabric softness and/or improved cleaning performance, including soil and stain removal, "dingy" fabric cleaning, whiteness maintenance. Without wishing to be bound by theory, it is believed that the deposition aid of the chemical entity binds to the fabric surface resulting in disruption of the fabric fibers. Soils and stains are so loosened from the fabric fibers, allowing the surfactant and protease to have an increased accessibility to the soils and stains. As a result of such increased accessibility, the activity of the surfactant and protease is enhanced, leading to an improved cleaning and/or fabric care performance. Furthermore, the disruption of the fabric fibers by the deposition aid increase the accessibility of the chemcial entity as well to the fabric and so enhances the activity of the attached benefit agents on the fabric surface, providing superior long-lasting perfume, sanitation, soil/stain removal, whiteness maintenance, fabric softness and/or fabric color care.

It has further been found that the addition of an amylase to the compositions of the present invention may further boost the cleaning performance. The amylase provides increased removal of carbohydrate/starch containing soils and stains, and starch from textile finishing treatment.

### THE CHEMICAL ENTITY

The laundry detergent and/or fabric care compositions of the present invention comprise as an essential component, a chemical entity. Such chemical entities comprise one or more deposition aid(s) having a high affinity for cellulose and one or more benefit agent(s), and potentially one or more linking region(s).

The chemical entities of the present invention are generally comprised in the laundry detergent and/or fabric care compositions at a level of from 0.00001 % to 50%, preferably 0.001% to 20%, more preferably from 0.1% to 10% by weight of the total composition.

### The Deposition Aid

The deposition aid according to the present invention is any material which has a high affinity for cellulose.

The deposition aid for the purpose of the present invention is a binding domain of a cellulose enzyme. In the context of the present invention the binding domain represents the binding site of an enzyme, defined as a sequence of amino acids which conserves the binding affinity of the binding site. Especially preferred is the binding site of cellulase, in particular the Cellulose Binding Domain (CBD).

For the purposes of the present invention, the deposition aid is indeed an "amino acid sequence comprising a Cellulose Binding Domain (CBD)", a term which is intended to indicate an amino acid sequence capable of effective binding of the cellulase to a cellulosic substrate (e.g., as described in P. Kraulis et al., Determination of the three-dimensional structure of the C terminal domain of cellobiohydrolase I from *Trichoderma reesei.* A study using nuclear magnetic resonance and hybrid distance geometry-dynamically simulated annealing. Biochemistry 28:7241-7257, 1989). The classification and properties of cellulose binding domains are presented in P. Tomme et al., in the symposium "Enzymatic degradation of insoluble polysaccharides" (ACS Symposium Series 618, edited by J.N. Saddler and M.H. Penner, ACS, 1995).

Cellulose-binding (and other carbohydrate-binding) domains are polypeptide amino acid sequences which occur as integral parts of large polypeptides or proteins consisting of two or more polypeptide amino acid sequence regions, especially in hydrolytic enzymes (hydrolases) which typically comprise a catalytic domain containing the active site for substrate hydrolysis and a carbohydrate-binding domain for binding to the carbohydrate substrate in question. Such enzymes can comprise more than one catalytic domain and one, two or three carbohydrate-binding domains, and they may further comprise one or more polypeptide amino acid sequence regions linking the carbohydrate-binding domain(s) with the catalytic domain(s), a region of the latter type usually being denoted a "linker" or "linking region".

Examples of hydrolytic enzymes comprising a cellulose-binding domain are cellulase, xylanases, mannanases, arabinofuranosidases, acetylesterases and chitinases. "Cellulose-binding domains" have also been found in algae, e.g. in the red alga *porphyra purpurea* in the form of a non-hydrolytic polysaccharide-binding protein [see P. Tomme et al., Cellulose-binding domains - Classification and Properties in Enzymatic Degradation of Insoluble Carbohydrates, John N. Saddler and Michael H. Penner (Eds.), ACS Symposium Series, No. 618 (1996)]. However, most of the known CBDs (which are classified and referred to by P. Tomme et al. (*op*. *cit.)* as "cellulose-binding domains") are derived from cellulases and xylanases.

In the present context, the term "cellulose-binding domain" is intended to be understood in the same manner as in the latter reference (P. Tomme et al., *op. cit.*) The P. Tomme et al. reference classifies more than 120 "cellulose-binding domains" into 10 families (I-X) which may have different functions or roles in connection with the mechanism of substrate binding. However, it is to be anticipated that new family representatives and additional families will appear in the future.

In proteins/polypeptides in which CBDs occur (e.g. enzymes, typically hydrolytic enzymes such as cellulases), a CBD may be located at the N or C terminus or at an internal position.

The part of a polypeptide or protein (e.g. hydrolytic enzyme) which constitutes a CBD *per se* typically consists of more than 30 and less than 250 amino acid residues. For example, those CBDs listed and classified in Family I in accordance with P. Tomme et al. (*op*. *cit.*) consist of 33-37 amino acid residues, those listed and classified in Family IIa consist of 95-108 amino acid residues, those listed and classified in Family VI consist of 85-92 amino acid residues, whilst one CBD (derived from a cellulase from *Clostridium thermocellum)* listed and classified in Family VII consists of 240 amino acid residues. Accordingly, the molecular weight of an amino acid sequence constituting a CBD per se will typically be in the range of from 4kD to 40kD, and usually below 35kD.

Cellulose binding domains can be produced by recombinant techniques as described in H. Stalbrand et al., Applied and Environmental Microbiology, Mar. 1995, pp. 1090-1097; E. Brun et al., (1995) Eur. J. Biochem. 231, pp. 142-148; J.B. Coutinho et al., (1992) Molecular Microbiology 6(9), pp. 1243-1252

In order to isolate a cellulose binding domain of, e.g. a cellulase, several genetic engineering approaches may be used. One method uses restriction enzyme to remove a portion of the gene and then to fuse the remaining gene-vector fragment in frame to obtain a mutated gene that encodes a protein truncated for a particular gene fragment. Another method involves the use of exonucleases such as Ba131 to systematically delete nucleotides either externally from the 5' and the 3' ends of the DNA or internally from a restricted gap within the gene. These gene-deletion methods result in a mutated gene encoding a shortened gene molecule whose expression product may then be evaluated for substrate-binding (e.g. cellulose-binding) ability. Appropriate substrates for evaluating the binding ability include cellulosic materials such as Avicel™ and cotton fibres. Other methods include the use of a selective or specific protease capable of cleaving a CBD, e.g. a terminal CBD, from the remainder of the polypeptide chain of the protein in question.

Preferred CBDs for the chemical entities of the present invention are selected from the group consisting of : CBDs CBHII from *Trichoderma reesei,* CBDs CenC, CenA and Cex from *Cellulomonas fimi,* CBD CBHI from *Trichoderma reesei,* CBD Cellulozome from *Clostridium cellulovorans,* CBD E3 from *Thermonospora fusca,* CBD-dimer from *Clostridium stecorarium* (NCIMB11754) XynA, CBD from *Bacillus agaradherens* (NCIMB40482), and/or CBD family 45 originating from the fungal *Humicola lnsolens* cellulase sold under the tradename "Carezyme" by Novo Nordisk A/S. Carezyme is an endoglucanase from family 45, derived from *Humicola insolens* DSM1800, having a molecular weight of 43kDa and exhibiting cellulolytic activity.
More preferred CBDs for the chemical entities of the present invention are the CBD CenC from *Cellulomonas fimi,* CBD Cellulozome from *Clostridium cellulovorans,* CBD family 45 from *Humicola Insolens,* and/or mixtures thereof.

### The linking region

The improved chemical entities of the present invention may further comprise one or more linking region(s). The term "linking region" is intended to indicate a region that adjoins the deposition aid and connects it to the chemical component(s). Suitable linking regions, if encompassed in the improved chemical entity of the present invention, are characterized by having one or more attachment point(s) for a chemical component and one or more moiety that will covalently bind to the deposition aid. Preferably, the linking regions of the present invention will encompass at least one attachment point to the deposition aid and more than one reactive group available to attach a chemical component, hereinafter referred to as a "polyreactive linking region". When present, this linking region can be achieved chemically or by recombinant techniques.

Suitable linking regions for the purpose of the present invention are :
1) Suitable are the polyethylene glycol derivatives described in the Shearwater polymers, Inc. catalog of January 1996, such as the nucleophilic PEGS, the carboxyl PEGs, the electrophilically activated PEGs, the sulfhydryl-selective PEGS, the heterofunctional PEGs, the biotin PEGS, the vinyl derivatives, the PEG silanes and the PEG phospholipids. In particular, suitable non-amino acid linking regions are the heterofunctional PEG, (X-PEG-Y) polymers from Shearwater such as PEG(NPC)2, PEG-(NH2)2, t-BOC-NH-PEG-NH2, t-BOC-NH-PEG-CO2NHS, OH-PEG-NH-tBOC, FMOC-NH-PEG-CO2NHS or PEG(NPC)₂ .MW 3400 from Sigma.
2) Other suitable linking regions are glutaric dialdehyde 50 wt% solution in water from Aldrich, disuccinimidyl suberate (DSS) form Sigma, γ-maleimidobutyric acid N-hydroxysuccinimide ester (GMBS) from Sigma, 1-ethyl-3-(3-dimethylaminopropyl)carbodiimide hydrochloride (EDC) from Sigma and dimethyl suberimidate hydrochloride (DMS) from Sigma, 1-ethyl-3-(3-dimethylaminopropyl) carbodiimide, N-ethyl-5-phenylisoaxolium-3-sulphonate, 1-cyclohexyl-3(2morpholinoethyl) carbodide metho-p-toluene sulphonate, N-ethoxycarbonyl-2-ethoxy 1,2, dihydroquinoline or glutaraldehyde.
3) Also suitable are the crosslinkers described in the 1999/2000 Pierce Products Catalogue from the Pierce Company, under the heading "Cross linking reagents : the SMPH, SMCC, LC-SMCC compounds, and preferably the Sulfo-KMUS compound.
   Preferred linking regions therefrom are PEG(NPC)2, (NH2)2-PEG, t-BOC-NH-PEG-NH2, MAL-PEG-NHS, VS-PEG-NHS polymers from Shearwater and/or the Sulfo-KMUS compound from Pierce.

Suitable polyreactive linking regions include polyacrylic acid/ maleic acid polymers, polyvinyl alcohol polymers and/or amine-containing compounds as described in co-pending application PCT/US98/20491 filed on September 30, 1998 by P&G, hereby incorporated by reference. Such amine-containing compounds include amino aryl derivatives, polyamines, substituted amines and amides, glucamines, dendrimers / chitosan saccharides, amine derivatives polysaccharides and peptidic polymers.
1) Poly acrylic acid/maleic acid : Suitable poly acrylic acid, maleic acid and/or mixtures thereof are those wherein one of the acid moiety is covalently bond to a NH2 group present in the amino acid sequence of the deposition aid. The other acid units are the reactive groups available as potential attachment groups for chemical components via their alcohol/amine groups.
2) Poly vinyl alcohol polymers. These polymers can still comprise or not the moiety containing the polymerisation initiator.
   - Without the acid moiety containing initiator of polymerisation, this polymer can be linked for example, to the reactive group of aspartic or glutamic acid present in the amino acid sequence of the deposition aid via an esterification reaction. The poly vinyl alcohol polymer does further comprise hydroxyl reactive groups available for attachment of acid/aldehyde containing chemical components.
   - With an acid moiety containing initiator of polymerisation, this acid moiety of the initiator of polymerisation of the polymer for example, can be linked for example, to a NH2 group present in the amino acid sequence of the deposition aid. The poly vinyl alcohol polymer does further comprise hydroxyl reactive groups available for attachment of acid/aldehyde containing chemical components.
3) Also suitable are amine-containing compounds as described WO 00/18897 filed on September 30, 1998 by P&G. hereby incorporated by reference and having the following general structure : B-(NH2)n; wherein B is a carrier material, and n is an index of value of at least 1.
   Preferred B carriers are inorganic or organic carriers. By "inorganic carrier", it is meant carrier which are non-or substantially non carbon based backbones.

Preferred primary amines, among the inorganic carriers, are those selected from mono or polymers or organic-organosilicon copolymers of amino derivatised organo silane, siloxane, silazane, alumane, aluminum siloxane, or aluminum silicate compounds. Typical examples of such carriers are: organosiloxanes with at least one primary amine moiety like the diaminoalkylsiloxane [H2NCH2(CH3) 2Si]O, or the organoaminosilane (C6H5) 3SiNH2 described in: Chemistry and Technology of Silicone, W. Noll, Academic Press Inc. 1998, London, pp 209, 106).

Preferred primary amines, among the organic carriers, are those selected from aminoaryl derivatives, polyamines, amino acids and derivatives thereof, substituted amines and amides, glucamines, dendrimers, the polyvinylamines with a MW of from 600-50K; amino substituted polyvinylalcohol with a MW ranging from 400-300,000; polyoxyethylene bis [amine]; polyoxyethylene bis [6-aminohexyl]; N,N'-bis-(3-aminopropyl)-1,3-propanediamine linear or branched; 1,4-bis-(3-aminopropyl) piperazine, and mixtures thereof.

Preferred aminoaryl derivatives are the amino-benzene derivatives including the alkyl esters of 4-amino benzoate compounds, and more preferably selected from ethyl-4-amino benzoate, phenylethyl-4-aminobenzoate, phenyl-4-aminobenzoate, 4-amino-N'-(3-aminopropyl)-benzamide, and mixtures thereof.

Polyamines suitable for use in the present invention are polyethyleneimines polymers, poly[oxy(methyl-1,2-ethanediyl)], -(2-aminomethylethyl)-(2-aminomethyl-ethoxy)- (= C.A.S No. 9046-10-0); poly[oxy(methyl-1,2-ethanediyl)], -hydro-)--(2-aminomethylethoxy)-, ether with 2-ethyl-2-(hydroxymethyl)-1,3-propanediol (= C.A.S. No. 39423-51-3); commercially available under the tradename Jeffamines T-403, D-230, D-400, D-2000; 2,2',2"-triaminotriethylamine; 2,2'-diamino-diethylamine; 3,3'-diamino-dipropylamine, 1,3 bis aminoethyl-cyclohexane commercially available from Mitsibushi and the C12 Sternamines commercially available from Clariant like the C12 Sternamin(propylenamine)n with n=3/4, and mixtures thereof. Preferred polyamines are polyethyleneimines commercially available under the tradename Lupasol like Lupasol FG, G20,wfv, PR8515, WF, FC, G20, G35, G100, HF, P, PS, SK, SNA.

Preferred substituted amines and amides for use herein are selected from nipecotamide, N-coco-1,3-propenediamine; N-oleyl-1,3-propenediamine; N-(tallow alkyl)-1,3-propenediamine; 1,4-diamino cyclohexane; 1,2-diaminocyclohexane; 1,12-diaminododecane, and mixtures thereof.

Other primary amine compounds suitable for use herein are the glucamines, preferably selected from 2,3,4,5,6-pentamethoxy-glucamine; 6-acetylglucamine, glucamine, and mixture thereof.

Also preferred compounds are the polyethylenimine and/or polypropylenimine dendrimers and the commercially available Starburst polyamidoamines (PAMAM) dendrimers, generation G0-G10 from Dendritech and the dendrimers Astromols, generation 1-5 from DSM being DiAminoButane PolyAmine DAB (PA)x dendrimers with x = 2nx4 and n being generally comprised between 0 and 4.

Still other preferred primary amine compounds are :
- polyvinylamines with a molecular weight (MW) ranging from 600, 1200, 3K, 20K, 25K or 50K;
- amino substituted polyvinylalcohol with a MW ranging from 400-300,000;
- polyoxyethylene bis [amine] available from e.g. Sigma;
- polyoxyethylene bis [6-aminohexyl] available from e.g. Sigma;
- N,N'-bis-(3-aminopropyl)-1,3-propanediamine linear or branched (TPTA)
- 1,4-bis-(3-aminopropyl) piperazine (BNPP).

The more preferred compounds are ethyl-4-amino benzoate, polyethyleneimine polymers commercially available under the tradename Lupasol like Lupasol FG, G20,wfv, PR8515, WF, FC, G20, G35, G100, HF, P, PS, SK, SNA; glucamine; the diaminobutane dendrimers Astramol, polyvinylamines with a MW ranging from 600, 1200, 3K, 20K, 25K or 50K; amino substituted polyvinylalcohol with a MW ranging from 400-300,000; polyoxyethylene bis [amine]; polyoxyethylene bis [6-aminohexyl]; N,N'-bis-(3-aminopropyl)-1,3-propanediamine linear or branched; 1,4-bis-(3-aminopropyl) piperazine, and mixture thereof. Most preferred primary amine compounds are selected from ethyl-4-amino benzoate, polyethyleneimine polymers commercially available under the tradename Lupasol like Lupasol FG, G20,wfv, PR8515, WF, FC, G20, G35, G100, HF, P, PS, SK, SNA; the diaminobutane dendrimers Astramol, N,N'-bis-(3-aminopropyl)-1,3-propanediamine linear or branched; 1,4-bis-(3-aminopropyl) piperazine, and mixtures thereof.Even most preferred compounds are those selected from ethyl-4-amino benzoate, polyethyleneimine polymers commercially available under the tradename Lupasol like Lupasol FG, G20,wfv, PR8515, WF, FC, G20, G35, G100, HF, P, PS, SK, SNA; N,N'-bis-(3-aminopropyl)-1,3-propanediamine linear or branched, 1,4-bis-(3-aminopropyl) piperazine, and mixtures thereof.

Most preferred polyreactive linking regions for use in the present invention are amino acids and their derivatives, especially ester and amide derivatives. These peptidic polymers are linked to the deposition aid via a peptidic linkage. More preferred compounds are those providing enhanced surface substantivity due to its structural feature.
Suitable amino acids have the following functionality of formula:

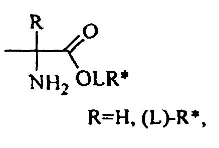

Suitable amino acids for use herein are selected tyrosine, tryptophane, lysine, glutamic acid, glutamine, aspartic acid, arginine, asparagine, phenylalanine, proline, glycine, serine, histidine, threonine, methionine, and mixture thereof. Homopolymers with the same amino acids or heteropolymers with different amino acids are suitable. For example, the amino acids Serine, Threonine and Tyrosine possess the reactive hydroxyl group, the Cysteine present a reactive SH group, the Asparagine and Glutamine amino acids possess a reactive amido group and the Lysine a reactive amino group. The linking will preferably be achieved on the Tyrosine, Cysteine or Lysine. Especially, the free NH2 group from a Lysine amino acid or of the terminal amino acid within the peptidic polymer is used as attachment points for chemicals containing aldehyde, ene one, ketone, or acid or halogene moeities
Also suitable compound are the amino acid derivatives selected from tyrosine ethylate, glycine methylate, tryptophane ethylate and mixture thereof,
These peptidic polymers can be attached to the amino acid sequence comprising a cellulose binding domain, by recombinant technology. An example of the recombinant technique describing the expression of an enzyme with the CBD of different origin is described in S. Karita et al., (1996) Journal of Fermentation and Bioengineering, Vol. 81, No. 6, pp. 553-556. The polyreactive linking region can comprise from 1 to 100 amino acid residues, in particular of from 2 to 40 amino acid residues, e.g. from 2 to 15 amino acid residues. It is preferred to use amino acids which are less favoured by the surrounding proteases. Indeed, any combination of amino acids can be selected to achieve maximal weight efficiency and protease stability. Suitable amino acid linking regions are the *Humicola insolens* family 45 cellulase linker, the NifA gene of *Klebsiella pneumoniae-*CiP linker, the *E. coli* OmpA gene-CiP linker, the E3 cellulase *Thermomonospora fusca* linker and the CenA cellulase linker; preferably the *Humicola insolens* family 45 cellulase linker and the E3 cellulase *Thermomonospora fusca* linker.

Depending on the intended activity of the attached chemical component, said chemical components can be linked permanently or temporarily to the deposition aid and/or linking region. Therefore, the present invention further encompasses chemical entities wherein the chemical component is linked to the deposition aid and/or linking region by a weak bond. Said weak bond is a bond which can be enzymatically cleaved, oxidized, cleaved by light radiation and/or hydrolysed during or after the wash/fabric care process in order to release the chemical component(s). Examples of weak bonds are Shiff-base or beta-amino-ketone bonds.

### The benefit agents

The chemical entity of the present invention comprises a benefit agent. The benefit agent is understood within the context of the present invention as any compound which gives a desirable effect on a fibre or fabric. The benefit agents encompassed in the chemical entity are preferably selected from perfumes, hygiene agents, insect control agent, fabric softening agents, soil release agents, bleaching agents, dye fixatives agents, brighteners, surfactants and/or mixtures thereof. More preferred benefit agents are the perfume agents, insect control agents, hygiene agents and/or bleaching agents.

In addition to these chemical entities, the compositions of the present invention can comprise the same benefit agents unmodified.

The benefit agent of the present invention may be encapsulated. Suitable encapsulating material includes starches, poly(vinylacetate), urea/formaldehyde condensate based materials. Especially suitable encapsulating materials are water soluble capsules which consist of a matrix of polysaccharide and polyhydroxy compounds such as described in GB 1,464,616. Other suitable water soluble encapsulating materials comprise dextrins derived from ungelatinized starch acid-esters of substituted dicarboxylic acids such as described in US 3,455,838. These acid-ester dextrins are preferably, prepared from such starches as waxy maize, waxy sorghum, sago, tapioca and potato. Suitable examples of said encapsulating materials include N-Lok manufactured by National Starch. The N-Lok encapsulating material consists of a modified maize starch and glucose. The starch is modified by adding monofunctional substituted groups such as octenyl succinic acid anhydride.

### Perfumes

A chemical entity suitable for the compositions of the present invention is a perfume entity wherein at least one benefit agent is a perfume compound linked to a deposition aid via a weak bond. It is believed that high substantivity of the perfume will be achieved by linking the perfume agents to a deposition aid.

Without wishing to be bound by theory, it is believed that the slow hydrolysis of the weak bond within the improved perfume entity will improve the release of the perfume. Indeed, after the wash or fabric care process, the weak bond will be hydrolysed and the perfume will be released. It has been surprisingly found that these perfume entities in the compositions of the present invention provide increased pleasing and long-lasting fragrance, through economic and effective means. Furthermore, without wishing to be bound by theory, it is believed that the binding of the perfume entities of the present invention to the fabric surface results in the disruption of the fabric fibers. The laundry soils and stains are loosend from the fabric fibers, and are rendered more accessible to the surfactant and protease. It has been found that the laundry detergent and/or fabric care compositions of the present invention, therefore provide long-lasting fragrance on laundered dry fabrics and improved cleaning performance.

Fully-formulated fragrance can be prepared using numerous known odorant ingredients of natural or synthetic origin. The range of the natural raw substances can embrace not only readily-volatile, but also moderately-volatile and slightly-volatile components and that of the synthetics can include representatives from practically all classes of fragrant substances, as will be evident from the following illustrative compilation. In this list of perfume ingredients, some are commercial names conventionally known to one skilled in the art, and also includes isomers. Such isomers are also suitable for use in the present invention. A typical disclosure of suitable ketone and/or aldehydes, traditionally used in perfumery, can be found in "perfume and Flavor Chemicals", Vol. I and II, S. Arctander, Allured Publishing, 1994, ISBN 0-931710-35-5. Preferred for the purpose of the present invention are the aldehydes or ketones based products.
- Natural products such as tree moss absolute, basil oil, citrus fruit oils (such as bergamot oil, mandarin oil, etc.), mastix absolute, myrtle oil, palmarosa oil, patchouli oil, petitgrain oil Paraguay, wormwood oil;
- Alcohols such as farnesol, geraniol, linalool, nerol, phenylethyl alcohol, rhodinol, cinnamic alcohol;
- Aldehydes such as citral, Helional™, alpha-hexyl-cinnamaldehyde, hydroxycitronellal, Lilial™ (p-tert.butyl-alpha -methyldihydrocinnamaldehyde), methylnonylacetaldehyde, 1-decanal, benzaldehyde, florhydral, 2,4-dimethyl-3-cyclohexen-1-carboxaldehyde; cis/trans-3,7-dimethyl-2,6-octadien-1-al; heliotropin; 2,4,6-trimethyl-3-cyclohexene-1-carboxaldehyde; 2,6-nonadienal; alpha-n-amyl cinnamic aldehyde, P.T. Bucinal, lyral, cymal, methyl nonyl acetaldehyde, hexanal, trans-2-hexenal, and mixture thereof;
- Ketones such as allylionone, alpha-ionone, beta -ionone, isoraldein (isomethyl-alpha -ionone), methylionone, Alpha Damascone, Delta Damascone, Iso Damascone, Carvone, Gamma-Methyl-lonone, Iso-E-Super, 2,4,4,7-Tetramethyl-oct-6-en-3-one, Benzyl Acetone, Beta Damascone, Damascenone, methyl dihydrojasmonate, methyl cedrylone, and mixtures thereof;
- Esters such as allyl phenoxyacetate, benzyl salicylate, cinnamyl propionate, citronellyl acetate, citronellyl ethoxolate, decyl acetate, dimethylbenzylcarbinyl acetate, dimethylbenzylcarbinyl butyrate, ethyl acetoacetate, ethyl acetylacetate, hexenyl isobutyrate, linalyl acetate, methyl dihydrojasmonate, styrallyl acetate, vetiveryl acetate, etc.;
- Lactones such as gamma-undecalactone, various components often used in perfumery, such as musk ketone, indole, p-menthane-8-thiol-3-one, and methyl-eugenol;
- Acetals and ketals include the well-known methyl and ethyl acetals and ketals, as well as acetals or ketals based on benzaldehyde, those comprising phenylethyl moieties, or more recently developed specialties such as those described in a United States Patent entitled "Acetals and Ketals of Oxo-Tetralins and Oxo-Indanes, see U.S. Pat. No. 5 ,084,440, issued January 28, 1992, assigned to Givaudan Corp. ;
- Recent synthetic specialties include the enol ethers of alkyl-substituted oxo-tetralins and oxo-indanes as described in U.S. Pat. 5,332,725, July 26, 1994, assigned to Givaudan; or Schiff Bases as described in U.S. Pat. 5,264,615, December 9, 1991, assigned to Givaudan.

When encompassed in the compositions of the present invention these perfume entities will be generally comprised at a level of from 0.001% to 20%, preferably from 0.005% to 5% by weight of the total composition.

### Hygiene agent

A further suitable chemical entity for the present invention is a hygiene entity wherein at least one benefit agent is a hygiene agent, linked to a deposition aid. The hygiene agent will be linked to the deposition aid and/or linking region via weak bond in order to release the active material upon time during or after the wash or fabric care process. Preferably, the hygiene agent comprises an hydroxyl, carboxyl or aldehyde reactive moiety.

It has been surprisingly found that such hygiene entities in the laundry detergent and/or fabric care compositions of the present invention, provide an extremely long lasting and very efficient control of the micro-organism growth on stored and weared fabrics. Without wishing to be bound by theory, it is believed that the hygiene entity binding to the fabric surface, increases the accessibility of the laundry soils and stains for surfactant and protease activity as well as for the hygiene agent, thereby allowing excellent control of micro-organism growth on stored and weared fabrics, as well as improved cleaning performance.

Sanitisation includes all positive effects obtained by the inhibition or reduction of microbial activity on fabrics and other surfaces, such as the prevention of malodour development and bacterial/fungal growth. For example, it provides prevention of malodour development on stored and weared fabrics. In particular, the composition of the invention will inhibit or at least reduce the bacterial and/or fungal development on moist fabric waiting for further laundry processing and thereby preventing the formation of malodour. The term hygiene agents herein encompasses fungicides and antimicrobials that when applied to fabric respectively prevent or reduce the growth of fungi or bacteria. The sanitisation benefits of the laundry detergent and/or fabric care compositions of the present invention can be evaluated by the Minimum Inhibitory Concentration (MIC) as described in Tuber. Lung. Dis. 1994 Aug; 75(4):286-90; J. Clin. Microbiol. 1994 May; 32(5):1261-7 and J. Clin. Microbiol. 1992 Oct; 30(10):2692-7.

Preferred antibacterial compounds are pentadecanol, cinamaidehyde, ionone, glutaraldehyde, citronellal. Other suitable antimicrobial compounds with an hydroxyl, carboxyl or aldehyde moiety are described in Parfums Cosmétiques Actualités No 125, Nov, 1995, 51-4. Other suitable antibacterial components are the Nerodol which can for example be linked to the carboxylic groups or succinic acid linked to the alcohol group of the deposition aid and/or linking region. A further example are the compounds Cipamaldehyde and/or Beta ionone which can form for example a Shiff base or β-amino ketone with the NH2 groups of the deposition aid and/or linking region.

Also suitable are the microbicidally active ingredients described in the handbook of Disinfectants and Antiseptics edited by J.M. Ascenzi and in WO97/4621 such as 2-hydroxydiphenyl ether, phenol derivatives, diphenyl compounds, benzyl alcohols, chlorhexidine, C12-14 alkylbetaines and C8-18 fatty acid amido alkylbetaines, amphoteric surfactants, trihalocarbanilides and quaternary ammonium salts, Also suitable are the cationic germicides described in EP 843 002 and in WO98/24314 and the antibacterial agents triclosan, triclocarban, DMDM hydantoin, piroctone olamine, zinc pyrithione, selenium disulfide, climnazole and 3-methyl-4-(1-methylethyl)phenol also therein described. Other examples of suitable fungicides are given in WO94/10286 (Henkel), CA943 429 (Unilever) and US3,426,024 (Henkel). Preferred antimicrobials are 2-thiocyanomethylthiobenzothiazole (Busan 30 WB ex Buckmann), butyl 4-hydroxybenzoate (Butyl Parabens ex Nipa Labs), propyl 4-hydroxybenzoate (Propyl Parabens ex Nipa Labs), Terpineol, Borneol, Fenchyl alcohol, trichlorocarbanilidem, Irgasan DP300 (2,4,4'-trichloro 2'hydroxydiphenylehter) and the higher homologues of hydroxybenzoate esters. Further examples of bactericides used in the hygiene agent entities of this invention include formaldehyde, 2-bromo-2-nitro-propane-1,3-diol sold by Inolex Chemicals, located in Philadelphia, Pennsylvania, under the trade name Bronopol®, and a mixture of 5-chloro-2-methyl-4-isothiazoline-3-one and 2-methyl-4-isothiazoline-3-one sold by Rohm and Haas Company under the trade name Kathon.

Further suitable hygiene agents include : 1- or 2-hexadecanol, 2-tetradecanol, 1-pentadecanol, 1-Undecanol, 2-dodecanol, 1-Tridecanol, nerolidiol, hinokitiol, tropolone, berberine, citronellic acid, Curcumin, 2-Mercaptopyridine N-oxide, Ellagic acid (dihydrate), 3-t-Butyl-5-methyl salicylic acid, 3-, 4- or 5-methyl salicylic acid, 1-nonanol, Decylalcohol, Cinamaldehyde, S- or R-Citronellal, Citronellol, Beta-ionone, Thujone, Coumarin and derivatives, Geraniol, Citral, Thymol, lso-butyl- or Isopropyl-quinoline, 2-butyl-5-methylphenol, 2-Mercapto-3-pyrodinol, Perillyl alcohol, 6-hydroxy-1,3-benzoxathiol-2-one, BOAT, (Iso)-Eugenol, Menth-1-en-9-ol, 2-t-Butyl-4-methylphenol, Kojic acid, Camphene, Carveol, Dihydroxycarvecol, lsojasmone, Methol, Cineol, Terpinol, Camphor, 2-t-Butyl-methyl phenol, 2-Tridecanone, Acetyl salicylic acid, Salicylaldoxime, Undecyclenic aldehyde, Nerol, 3,5,5-trimethyl-1-hexanol, Adipic acid, Thiosalicylic acid, OH-Benzoic acid, 2-Methylbenzothiazole, 2-Aminobenzothiazole, Caryophyllene, Allyl-isocyanate, Carvone, Alpha-pinene, Salicylic acidfrole, Alpha-ionone, 2OH- or 3OH-Phenethyl alcohol, Trimethoxy BP, Undecylic aldehyde, Cineole, Anisaldehyde, Bornyl acetate, Salicylhydroxamic, Benzofuran Car., Syringaldehyde.

Preferably, the levels of the hygiene entity should be such that they prevent bacterial and fungi growth on fabrics, rather than merely preventing growth within the laundry detergent and/or fabric care compositions per se. When encompassed in the compositions of the present invention, these hygiene entities will be generally comprised at a level of from 0.00001% to 20%, preferably from 0.00001% to 5% by weight of the total composition.

### Insect Control agent

Further suitable chemical entities for the compositions of the present invention are insect control entities wherein at least one benefit agent is an insect control agent, linked to a deposition aid.

It has surprisingly been determined that such insect control entities in the laundry detergent and/or fabric care compositions of the present invention provide a much longer lasting insect control by the slow release of the insect control agent. Indeed, the binding of the insect control entity to the fabric surface results in fabric fiber disruption and thus the loosening of soils and stains and in better accessibility for the surfactant and protease, as well as for the insect control agent. Thereby, the laundry detergent and/or fabric care compositions of the present invention combining such insect control entities with a surfactant and a protease, provide enchanced insect control performance and improved cleaning performance.

Such insect control agents are linked to the deposition aid and/or linking region preferably via a weak bond in order to release upon time the active material. For example, these materials can be linked to the NH2 group present in the deposition aid and/or linking region via a Shiff base or Michael reaction.

The term insect control agent refers to both insecticides and insect repellents either individually or as mixtures. Examples of insect repellents can be found in Kirk-Othmer Encyclopedia of Chemical technology, Fourth edition, volume 13, pages 474 to 478. Suitable insect repellents include aldehyde based compounds such as Citronellal and Rotundial, ene one based compounds such as Butopyronoxyl (Indalone TM), benzyl benzoate, bioallethrin and dimethrin, N,N-diethyl toluamide ("DEET"), N,N-diethyl benzamide, p-menthane-3,8-diol, 1S,3S,4S,6S-carene-3,4diol (Sumitomo - US5,130,136), 1-piperidinecarboxylic acid, 2-(2-hydroxyethyl)-, 1-methylpropylester, 1-(3cyclohexen-1-yl carbonyl)-2methylpiperidine, 1-(3-cyclohexen-1-yl carbonmyl) piperidine, N,N-diethyl mandelamide, isopulegol hydrate, ethyl-3(N-butyl-N-acetyl) aminopropionate, diisopropyladipate, α-biasal, psearmint oil, benzyl alcohol, N,N-diethylphenylacetamide, vitamin E, citronella oil, coconut oil, cedar oil, geraniol, lemon grass oil, thyme oil, reosemary oil, mint oil, geranium oil, eugenol, 3-acetyl-2-(2-,6-dimethyl-5-heptenyl) oxazolidine, (2-hydroxymethylcyclohexyl)acetic acid lactone and eucalyptol. Other insect control agents are based on pyrethroid insecticides, in particular 3-phenoxybenzyl-DL-cis, trans-3-(2,2-dichlorovinyl)-2,2-dimethylcyclopropane-carboxylate (permethrin). WO98/17772 describes the insect repellent agent 3-(N-butylacetamino) ethyl propionate for use in detergents. Preferred insect repellents are aldehyde based compounds such as Citronellal and Rotundial.

When encompassed in the compositions of the present invention, these insect control entities are generally comprised at a level of from 0.1% to 40%, preferably from 0.1 % to 10% by weight of the total composition.

### Bleaching agent

A further suitable chemical entity for the present invention is a bleaching entity wherein at least one benefit agent is a bleaching agent, linked to a deposition aid. Said bleaching agent can be selected from hydrophilic bleach activators, hydrophobic bleach activators, metal catalysts and/or photoactivated bleaches.

It has been surprisingly found that the bleaching action with such bleaching entities within the laundry detergent and/or fabric care compositions of the present invention, results in improved and increased stain/soil bleaching/removal and whiteness maintenance. Without wishing to be bound by theory, it is believed that improved stain/soil bleaching/removal and whiteness maintenance results in the generation of oxygen radicals, peracetic acid or peracid perhydrolysis occurring very close to the fabric surface. Said peracetic acid or peracid is less dilute in the wash solution due to the bleach entity of the present invention being very efficiently deposited onto the fabric and thereby improving the bleaching action on the fabric surface. Furthermore, the bleaching action from the bleaching entity may also lead to laundry soils / stains becoming solubilized due to the bleach activity oxidizing a-polar groups into more polar groups. The soluble laundry soils and stains are rendered more accessible to the surfactant and protease by the binding of the chemical entity, thereby resulting in improved cleaning performance, including soil and stain removal, dingy cleaning and/or whiteness maintenance.

Preferably, the bleach activator is linked to the deposition aid via a linking region, more preferably via a polyreactive linking region. Without wishing to be bound by theory, it is believed that the presence of such a linking region provides flexibility of movement to the bleach activator therefore enabling it to perform more efficiently its bleaching action on the fabric fibers.
1) Hydrophilic bleach activator : For example, Tetra Acetyl Ethylene Diamine (TAED) is a current bleach activator used in laundry detergent and forms peracetic acid in presence of H2O2. It is known that the two NH2 groups of the Ethylene Diamine are diacetylated to form TAED. Without wishing to be bound by theory, it is believed that the diacetylation of the free NH2 groups of the deposition aid and/or linking region, forms a substantive fabric hydrophilic bleach activator. Hydrophilic bleach activator of the TAED type can be linked for example via a diacetylation reaction to any NH2 groups of the deposition aid and/or linking region of the present invention.
2) Hydrophobic bleach activators such as Nonanoyl benzene sulphonate, are current bleach activators used in laundry detergent. It has been surprisingly found that the phenolic moiety can be replaced by the one present in the tyrosine amino acid present in the deposition aid and/or linking region. Active hydrophobic bleach activator precursors such as nonanyl unit can be linked to the phenol group of an amino acid such as tyrosine present in the peptidic deposition aid and/or linking region. Similarly, if there is no phenol entity available in the deposition aid and/or linking region, a phenol unit can be linked to a group NH2 in this deposition aid and/or linking region. Hydrophobic bleach activators in general can be linked to the deposition aid and/or linking region for example by a reaction on NH2 groups.
   Other examples of suitable hydrophobic bleach activators are nonanoyloxybenzene-sulfonate (NOBS, described in US 4,412,934), 3,5,-trimethylhexanoloxybenzenesulfonate (ISONOBS, described in EP 120,591) or pentaacetylglucose (PAG) or Phenolsulfonate ester of N-nonanoyl-6-aminocaproic acid (NACA-OBS, described in WO94/28106), which are perhydrolyzed to form a peracid as the active bleaching species, leading to improved bleaching effect.
   Also suitable bleach activators are the activators based on a caprolactam leaving group such as benzoyl caprolactam and quaternary ammonium hexanoyl caprolactam; the imides activators such as N-nonanoyl-N-methyl acetamide and unsymetrical acyclic imide bleach activator of the following formula as disclosed in the Procter & Gamble co-pending patent application. WO 98/04664 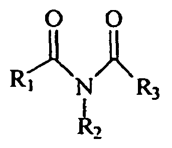 wherein R₁ is a C₇-C₁₃ linear or branched chain saturated or unsaturated alkyl group, R₂ is a C₁-C₈, linear or branched chain saturated or unsaturated alkyl group and R₃ is a C₁-C₄ linear or branched chain saturated or unsaturated alkyl group. These bleach activators can for example be linked to any NH2 groups of the polyreative linking region.
   Also suitable are the pre-fromed peracid such as nonytamido peroxy adipic acid and N,N-phtaloylaminoperoxy caproic acid and the Di-acyl peroxides such as dobenzoyl peroxide.
3) Metal catalysts : A third type of bleach activator that can be encompassed in the bleaching entity of the present invention, are the below described metal catalysts. For example, these catalysts can be linked via their capping cyclo azo moieties to NH2 groups of the deposition aid and/or linking region. Examples of Metal-containing catalysts for use in bleach compositions, include cobalt-containing catalysts such as Pentaamine acetate cobalt(III) salts and manganese-containing catalysts such as those described in EPA 549 271; EPA 549 272; EPA 458 397; US 5,246,621; EPA 458 398; US 5,194,416 and US 5,114,611. Bleaching composition comprising a peroxy compound, a manganese-containing bleach catalyst and a chelating agent is described in the patent application No 94870206.3.
4) Photoactivated bleach : Another group of suitable bleach activators that can be encompassed in the bleaching entity of the present invention, are the photoactivated bleaches. For example, the linking can be achieved between the NH2 groups of and the benzyls groups of these photoactivated bleaches, activated via a preliminary bromination.
   Suitable photoactivated bleaching agents are the sulfonated zinc and/or aluminum phthalocyanines. These materials can be deposited upon the substrate during the washing process. Upon irradiation with light, in the presence of oxygen, such as by hanging clothes out to dry in the daylight, the sulfonated zinc phthalocyanine is activated and, consequently, the substrate is bleached. Preferred zinc phthalocyanine and a photoactivated bleaching process are described in U.S. Patent 4,033,718. Typically, the compositions herein will contain 0.025% to 1.25%, by weight of sulfonated zinc phthalocyanine.

In addition to this bleaching entity, the compositions of the present invention can further comprise bleaching species such as hydrogen peroxide, PB1, PB4 and percarbonate with a particle size of 400-800 microns, typically be present at levels of from 1 % to 25%.

When encompassed in the compositions of the present invention, these bleaching entities will be generally comprised at a level of from 0.001% to 40%, preferably from 0.1% to 10% by weight of the total composition.

### Fabric softening agents

For the purpose of the present invention, further suitable chemical entities are fabric softening entities wherein at least one benefit agent is a fabric softening agent, linked to a deposition aid. It has been surprisingly found that such fabric softening entities within the laundry detergent and/or fabric care compositions of the present invention, provide anti-wrinkling, anti-bobbling and anti-shrinkage properties, as well as fabric softness. Without wishing to be bound by theory, it is believed that the improved fabric care and/or cleaning performance results from improved softening or soil/stain removal activity by the surfactant and protease due to the fabric softening entity providing an increased accessibility to soils and stains. In addition, the binding of the deposition aid of the fabric softening entity to the fabric results in the loosening of the soils and stains and in increased accessibility of the fabric softening entity as well as of the Ca⁺⁺ salts to the fabric surface. These Ca⁺⁺ salts are known to give a "harsh" feeling to the fabric. Therefore, the combined activity the fabric softening entity, the protease and surfactant, for example a softener cationic surfactant, leads to improved cleaning and/or fabric care including including anti-wrinkling, anti-bobbling and anti-shrinkage properties, as well as fabric softness.

Indeed, recent years, consumer desirability for durable press fabric garments, particularly cotton fabric garments, has risen. Durable press garments include those garments which resist wrinkling of the fabric both during wear and during the laundering process. Durable press garments can greatly decrease the hand work associated with laundering by eliminating ironing sometimes necessary to prevent wrinkling of the garment. However, in most commercially available durable press fabrics, the fabric's ability to resist wrinkling is reduced over time as the garment is repeatedly worn and laundered. It has been found that the fabric softening entities of the present invention provide excellent anti-wrinkling, anti-bobbling and anti-shrinkage properties, as well as fabric softness properties due to effective deposition of the fabric softening entity on the fabric with the combined effect of the surfactant and protease.

Suitable fabric softening agents are dialkyl units that can be linked for example, by dialkylation of the NH2 groups comprised in the deposition aid and/or linking region. Preferably such fabric softening entities will not comprise a weak bond.

Dialkyl units suitable for the compositions of the present invention can be extracted from the following cationic softening surfactants currently used in the laundry detergent and/or fabric care context.
The alkyl, or alkenyl chain will contain at least 11 carbon atoms, preferably at least 16 carbon atoms. The chain may be straight or branched. Specific examples of the alkyl or alkenyl chains herein include :
1) N,N-di(tallowyl-oxy-ethyl);
2) N,N-di(2-tallowyloxy-2-oxo-ethyl);
3) N,N-di(2-tallowyloxyethylcarbonyloxyethyl);
4)N-(2-tallowoyloxy-2-ethyl)-N-(2-tallowyloxy-2-oxo-ethyl);
5) N-(2-tallowyloxy-2-oxoethyl)-N-(tallowyl); and
6) 1,2-ditallowyloxy;
and mixtures of any of the above materials.
Preferred alkyl chains for the purpose of the present invention N,N-di(tallowoyloxy-ethyl), where the tallow chains are at least partially unsaturated.

Other suitable fabric softening agents include quaternary ammonium softening compounds having a solubility in water at pH2.5 and 20°C of less than 10g/l. It is particularly advantageous if the fabric softening agent is a quaternary ammonium compound in which at least one long chain alkyl group is connected to the quaternary ammonium compound via at least one ester link. Suitable cationic softeners are described in US4,137,180 (Naik) and WO93/23510.

Also suitable as softening components are clay or silicone.
Suitable clays include a three layered smectite clay, preferably having a cationic exchange capacity as described in GB 1,400,898 and in USP 5,019,292. Especially preferred are clays which are 2:1 layer phyllosilicates possessing a lattice charge efficiency in the range of 0.2 to 0.4g equivalent per half unit cell as described in EP 350 288 (Unilever).
Also encompassed in the present invention are any polymeric lubricant suitable for softening a fabric. These include silicone and in particular those described in GB1,549,180; EP 459 821 (Unilever) and EP 459 822 (Unilever).

When encompassed in the present invention, these improved fabric softening entities will be genrally comprised at a level of from 0.5% to 50%, preferably from 1% to 30%, more preferably from 2% to 15% by weight of the total composition.

### Dye fixative agents

Further suitable chemical entities suitable for the present invention are dye fixative entities wherein at least one benefit agent is a dye fixative agent, linked to a deposition aid. It has been surprisingly found that such dye fixative entities in the laundry detergent and/or fabric care compositions of the present invention, provide enchanced anti-wear properties and colour appearance.

indeed, coloured garments have a tendency to wear and show colour appearance losses. A portion of this wear and colour appearance loss may be attributed to abrasion in the laundering process, particularly in automatic washing machines and automatic laundry dryers. It has been found that the laundry detergent and/or fabric care compositions of the present invention combining such a dye fixative entity with a surfactant and a protease provide reduced wear and colour appearance loss, and improved cleaning performance. Without wishing to be bound by theory, it is believed that the reduced wear and colour appearance loss, and improved cleaning performance results from the increased accessibility of laundry soils / stains and fabric fibers due to binding of the dye fixative entity to the fabric surface.

Dye fixative agents are well-known, commercially available materials which are designed to improve the appearance of dyed fabric by minimising the loss of dye from fabrics due to washing. Many dye fixatives are cationic, and are based on various quatemized or otherwise cationically charged organic nitrogen compounds. Fixatives are available under various trade names from several suppliers. Representative examples include: CROSCOLOR PMF (July 1981, Code No. 7894) and CROSCOLOR NOFF (January 1988, Code No. 8544) from Crosfield; INDOSOL E-50 (February 27, 1984, Ref. No. 6008.35.84; polyethyleneamine-based) from Sandoz; SANDOFIX TPS, which is also available from Sandoz and is a preferred polycationic fixative for use herein and SANDOFIX SWE (cationic resinous compound), REWIN SRF, REWIN SRF-O and REWIN DWR from CHT-Beitlich GMBH. Other cationic dye fixative agents are described in "Aftertreatments for improving the fastness of dyes on textile fibres" by Christopher C. Cook (REV. PROG. COLORATION Vol. 12, 1982).

Other dye fixative agents suitable for use in the present invention are ammonium compounds such as fatty acid - diamine condensates e.g. the hydrochloride, acetate, metosulphate and benzyl hydrochloride of cleyldiethyl aminoethylamide, oleylmethyl-diethylenediaminemethsulphate, monostearyl-ethylene diaminotrimethylammonium methosulphate and oxidised products of tertiary amines; derivatives of polymeric alkyldiamines, polyamine-cyanuric chloride condensates and aminated glycerol dichlorohydrins.

When encompassed in the compositions of the present invention, these dye fixative entities are generally comprised at a level of from 0.01% to 30%, preferably from 0.1 % to 15% by weight of the total composition.

### Soil release agents

A further suitable chemical entity for the compositions of the present invention is a soil release entity wherein at least one benefit agent is a soil release agent, linked to a deposition aid. It has been surprisingly found that such soil release agents in the laundry detergent and/or fabric care compositions of the present invention, provide better deposition of the soil release agent onto the fabric and therefore better soil release performance. Without wishing to be bound by theory, it is beleived that the interaction of the soil release entity with the fabric surface, results in increased accessibility of the soils and/or stains, thereby allowing a significantly improved cleaning performance by the surfactant, protease and soil removal agent.

Suitable soil release agents for use in the present invention include ethylene glycols oligomers, polyethylene glycol and derivatives such as transesterified polyethylene glycols, and propylene polyoxy ethylene. Other suitable soil release agents useful in compositions of the present invention are conventionally copolymers or terpolymers of terephthalic acid with ethylene glycol and/or propylene glycol units in various arrangements. Examples of such polymers are disclosed in the commonly assigned US Patent Nos. 4116885 and 4711730 and European Published Patent Application No. 0 272 033. A particular preferred polymer in accordance with EP-A-0 272 033 has the formula

(CH₃(PEG)₄₃)_{0.75}(POH)_{0.25}[T-PO)_{2.8}(T-PEG)_{0.4}]T(PO-H)_{0.25}((PEG)₄₃CH₃)_{0.75}

where PEG is -(OC₂H₄)O-,PO is (OC₃H₆O) and T is (pcOC₆H₄CO).

Also very useful are modified polyesters as random copolymers of dimethyl terephthalate, dimethyl sulfoisophthalate, ethylene glycol and 1-2 propane diol, the end groups consisting primarily of sulphobenzoate and secondarily of mono esters of ethylene glycol and/or propane-diol. The target is to obtain a polymer capped at both end by sulphobenzoate groups, "primarily", in the present context most of said copolymers herein will be end-capped by sulphobenzoate groups. However, some copolymers will be less than fully capped, and therefore their end groups may consist of monoester of ethylene glycol and/or propane 1-2 diol, thereof consist "secondarily" of such species.
The selected polyesters herein contain 46% by weight of dimethyl terephthalic acid, 16% by weight of propane-1.2 diol, 10% by weight ethylene glycol 13% by weight of dimethyl sulfobenzoic acid and 15% by weight of sulfoisophthalic acid, and have a molecular weight of 3.000. The polyesters and their method of preparation are described in detail in EPA 311 342.

Other soil release agents currently used in the detergent context are those of U.S. 4,968,451, November 6, 1990 to J.J. Scheibel and E.P. Gosselink: such ester oligomers can be prepared by (a) ethoxylating allyl alcohol, (b) reacting the product of (a) with dimethyl terephthalate ("DMT") and 1,2-propylene glycol ("PG") in a two-stage transesterification/oligomerization procedure and (c) reacting the product of (b) with sodium metabisulfite in water; the nonionic end-capped 1,2-propylene/polyoxyethylene terephthalate polyesters of U.S. 4,711,730, December 8, 1987 to Gosselink et al, for example those produced by transesterification/oligomerization of poly(ethyleneglycol) methyl ether, DMT, PG and poly(ethyleneglycol) ("PEG"); the partly- and fully- anionic-end-capped oligomeric esters of U.S. 4,721,580, January 26, 1988 to Gosselink, such as oligomers from ethylene glycol ("EG"), PG, DMT and Na-3,6-dioxa-8-hydroxyoctanesulfonate; the nonionic-capped block polyester oligomeric compounds of U.S. 4,702,857, October 27, 1987 to Gosselink, for example produced from DMT, Me-capped PEG and EG and/or PG, or a combination of DMT, EG and/or PG, Me-capped PEG and Na-dimethyl-5-sulfoisophthalate; and the anionic, especially sulfoaroyl, end-capped terephthalate esters of U.S. 4,877,896, October 31, 1989 to Maldonado, Gosselink et al, the latter being typical of SRA's useful in both laundry and fabric conditioning products, an example being an ester composition made from m-sulfobenzoic acid monosodium salt, PG and DMT optionally but preferably further comprising added PEG, e.g., PEG 3400. Another preferred soil release agent is a sulfonated end-capped type described in US 5,415,807.

When encompassed in the compositions of the present invention, these soil release entities are generally comprised at a level of from 0.01% to 20%, preferably from 0.1 % to 5% by weight of the total composition.

### Brightener agents

Also suitable for the purpose of the present invention, is a brightener entity wherein at least one benefit agent is a brightener, linked to a deposition aid. Without wishing to be bound by theory, it is believed that such brightener entities within the laundry detergent and/or fabric care compositions of the present invention, provides better whiteness maintenance performance. Without wishing to be bound by theory, it is beleived that the binding of the brightener entities disrupts the fabric surface, loosens the laundry soils and stains therefrom, allowing better accessibility for better surfactant, protease and brightener performance. Therefore, the laundry detergent and/or fabric care compositions of the present invention combining such brigthener entities with a surfactant and a protease, provide superior whiteness maintenance performance and improved cleaning performance.

Suitable brighteners for compositions of the present invention are :
- Carbocycles types of compounds such as distyrylbenzenes, distyrylbiphenyls and divinylstibenes,
- Triazinylaminostilbenes,
- Stilbenyl-2H-triazoles such as stilbenyl-2H-naphtol[1,2-d]triazoles and bis(1,2,3-triazol-2-yl)stilbenes,
- Benzoxazoles such as stilbenylbenzoxazoles and bis(benzoxazoles),
- Furans, Benzo[b]furnas and Benzimidazoles such as bis(benzo[b]furan-2-yl)biphenyls and cationic benzimidazoles,
- 1,3-Diphenyl-2-pyrazolines
- Coumarins
- Naphtalimides
- 1,3,5-triazin-2-yl-Derivatives.
The brightener agents, for example of the Coumarin type, can be attached to the NH2 group or to an amido group from the deposition aid and/or linking region.

Preferred are the bleach-stable brighteners such as 1,4-di(2-methylaminostyryl)benzene.

Other suitable brighteners are the hydrophilic optical brighteners having the structural formula: 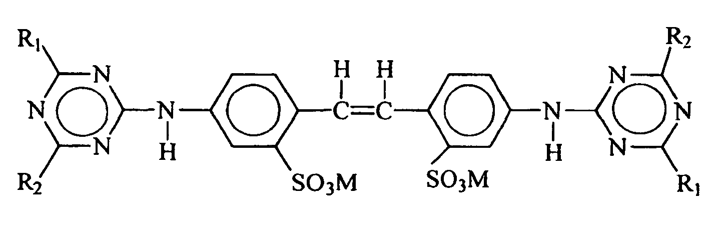 wherein R₁ is selected from anilino, N-2-bis-hydroxyethyl and NH-2-hydroxyethyl; R₂ is selected from N-2-bis-hydroxyethyl, N-2-hydroxyethyl-N-methylamino, morphilino, chloro and amino; and M is a salt-forming cation such as sodium or potassium.
- When in the above formula, R₁ is anilino, R₂ is N-2-bis-hydroxyethyl and M is a cation such as sodium, the brightener is 4,4',-bis[(4-anilino-6-(N-2-bis-hydroxyethyl)-s-triazine-2-yl)amino]-2,2'-stilbenedisulfonic acid and disodium salt. This particular brightener species is commercially marketed under the tradename Tinopal-UNPA-GX by Ciba-Geigy Corporation. Tinopal-UNPA-GX is the preferred hydrophilic optical brightener useful in the rinse added compositions herein.
- When in the above formula, R₁ is anilino, R₂ is N-2-hydroxyethyl-N-2-methylamino and M is a cation such as sodium, the brightener is 4,4'-bis[(4-anilino-6-(N-2-hydroxyethyl-N-methylamino)-s-triazine-2-yl)amino]2,2'-stilbenedisulfonic acid disodium salt. This particular brightener species is commercially marketed under the tradename Tinopal 5BM-GX by Ciba-Geigy Corporation.
- When in the above formula, R₁ is anilino, R₂ is morphilino and M is a cation such as sodium, the brightener is 4,4'-bis[(4-anilino-6-morphilino-s-triazine-2-yl)amino]2,2'-stilbenedisulfonic acid, sodium salt. This particular brightener species is commercially marketed under the tradename Tinopal AMS-GX by Ciba Geigy Corporation.

Other suitable optical brighteners are anionic in character, examples of which are disodium 4,4'-bis-(2-diethanolamino-4-anilino -s- triazin-6-ylamino)stilbene-2:2' disulphonate, disodium 4, - 4'-bis-(2-morpholino-4-anilino-s-triazin-6-ylaminostilbene-2:2' - disulphonate, disodium 4,4' - bis-(2,4-dianilino-s-triazin-6-ylamino)stilbene-2:2' - disulphonate, monosodium 4',4" -bis-(2,4-dianilino-s-triazin-6 ylamino)stilbene-2-sulphonate, disodium 4,4' -bis-(2-anilino-4-(N-methyl-N-2-hydroxyethylamino)-s-triazin-6-ylamino)stilbene-2,2' - disulphonate, di-sodium 4,4' -bis-(4-phenyl-2,1,3-triazol-2-yl)-stilbene-2,2' disulphonate, di-so-dium 4,4'bis(2-anilino-4-(1-methyl-2-hydroxyethylamino)-s-triazin-6- ylami-no)stilbene-2,2'disulphonate, sodium 2(stilbyl-4"-(naphtho-1',2':4,5)-1,2,3 - triazole-2"-sulphonate and 4,4'-bis(2-sulphostyryl)biphenyl. Highly preferred brighteners are the specific brighteners of copending European Patent application No. 95201943.8.

It has been surprisingly found that various brighteners not currently used in the detergent field could also be used in the present invention thanks to their increased fabric substantivity, in particular during a softening-through-the-wash process. Preferably, such brightener entities will not comprise a weak bond.

When encompassed in the compositions of the present invention, these brightnener entities will be generally comprised at a level from 0.001% to 10%, preferably from 0.005% to 3.5% by weight of the total composition.

### Other suitable benefit agents

Also suitable as chemical entities for the compositions of the present invention are latex and resin entities wherein at least one benefit agent is a latex or resin agent, linked to a deposition aid. Latex is defined as a material suitable for refining the drape of fabric. Resins prevent the formation of pills on the fabrics.

Without wishing to be bound by theory, it is believed that these chemical entities upon binding to the fabric surface increase the accessibility of laundry soils and stains, allowing better accessibility for better surfactant, protease, latex and resin performance. Therefore, the laundry detergent and/or fabric care compositions of the present invention, comprising these chemical entities with a surfactant and a protease, provide refining of fabric drape, prevention of pill formation, and improved cleaning performance.

Suitable latex materials include a polyvinylacetate homopolymer such as 9802 (Vinamul). Suitable resins are Knittex BE from Ciba-Geigy or silicas such as Crosanol NS from Crosfiled.

### THE SURFACTANT

The second essential element of the laundry detergent and/or fabric care compositions according to the present invention, is a surfactant selected from nonionic, anionic, cationic, ampholytic, zwitterionic, semi-polar surfactants and/or mixtures thereof.

It has been surprisingly found that the laundry detergent and/or fabric care compositions of the present invention comprising a chemical entity, a surfactant and a protease, achieve improved cleaning and/or fabric care performance. Without wishing to be bound by theory, it is believed that the binding of chemical entity to the fabric surface disrupts the fabric fibers, resulting in the loosening of the laundry soils and stains and therefore increased accessibility to laundry soils and stains, enabling the surfactant to efficiently remove such soils and stains. It further increase the accessibility of the chemical entity to the fabric fibre and so enhance its performance. Therefore, such laundry detergent and/or fabric care compositions provide improved cleaning performance, including soil and stain removal, "dingy" fabric cleaning, whiteness maintenance. Moreover, such increased accessibility of the fabric fibers, enables the surfactant, especially a cationic surfactant, to provide improved fabric care benefits, including anti-wrinkling, anti-bobbling and anti-shrinkage properties, as well as fabric softness.

Surfactants are typically present at a level of from 0.1 % to 60%, preferably from 1% to 35%, more preferably from 1% to 30% by weight of the total composition of the present invention.

A preferred surfactant system for the compositions of the present invention is a surfactant system comprising a mixture of nonionic and anionic surfactants.

Such a surfactant system will preferably comprise a weight ratio of nonionic to anionic of at least 1 : 10. As described below, preferred nonionic surfactants are selected from polyethylene oxide condensates of alkyl phenols, polyhydroxy fatty acid amide and/or mixtures thereof and preferred anionic surfactants are selected from linear alkyl benzene sulfonate, alkyl ethoxylates sulfates, alkyl sulfates, mid-chain-branched anionics and/or mixtures thereof.

The surfactant systems of the laundry detergent and fabric care compositions of the present invention will preferably further comprise a cationic surfactant. Suitable cationic surfactants are detergent cationics providing fabric cleaning benefits, and softening cationics providing fabric softness. Preferred cationic surfactants for the purposes of the present invention are the "detergent" cationic surfactants. By "detergent" cationic surfactants is meant a cationic surfactant having detersive properties. Preferred detergent cationic surfactants are selected from : coconut trimethyl ammonium chloride or bromide, preferably coconut methyl dihydroxyethyl ammonium chloride or bromide, most preferably decyl dimethyl hydroxyethyl ammonium chloride or bromide, and/or mixtures thereof.
The laundry detergent and/or fabric care compositions of the present invention preferably comprise said cationic surfactants at levels of no more than 5%, preferably from 1% to 2% by weight of the composition.

Nonionic surfactants : Suitable nonionic surfactants for the surfactant systems of the present invention, are polyethylene, polypropylene, and polybutylene oxide condensates of alkyl phenols with the polyethylene oxide condensates being preferred. These compounds include the condensation products of alkyl phenols having an alkyl group containing from 6 to 14 carbon atoms, preferably from 8 to 14 carbon atoms, in either a straight-chain or branched-chain configuration with the alkylene oxide. In a preferred embodiment, the ethylene oxide is present in an amount equal to from 2 to 25 moles, more preferably from 3 to 15 moles, of ethylene oxide per mole of alkyl phenol. Commercially available nonionic surfactants of this type include lgepal™ CO-630, marketed by the GAF Corporation; and Triton™ X-45, X-114, X-100 and X-102, all marketed by the Rohm & Haas Company. These surfactants are commonly referred to as alkylphenol alkoxylates (e.g., alkyl phenol ethoxylates).

The condensation products of primary and secondary aliphatic alcohols with from 1 to 25 moles of ethylene oxide are suitable for use as the nonionic surfactant of the surfactant systems of the present invention. The alkyl chain of the aliphatic alcohol can either be straight or branched, primary or secondary, and generally contains from 8 to 22 carbon atoms. Preferred are the condensation products of alcohols having an alkyl group containing from 8 to 20 carbon atoms, more preferably from 10 to 18 carbon atoms, with from 2 to 10 moles of ethylene oxide per mole of alcohol. 2 to 7 moles of ethylene oxide and most preferably from 2 to 5 moles of ethylene oxide per mole of alcohol are present in said condensation products. Examples of commercially available nonionic surfactants of this type include Tergitol™ 15-S-9 (the condensation product of C₁₁-C₁₅ linear alcohol with 9 moles ethylene oxide), Tergitol™ 24-L-6 NMW (the condensation product of C₁₂-C₁₄ primary alcohol with 6 moles ethylene oxide with a narrow molecular weight distribution), both marketed by Union Carbide Corporation; Neodol™ 45-9 (the condensation product of C₁₄-C₁₅ linear alcohol with 9 moles of ethylene oxide), Neodol™ 23-3 (the condensation product of C₁₂-C₁₃ linear alcohol with 3.0 moles of ethylene oxide), Neodol™ 45-7 (the condensation product of C₁₄₋C₁₅ linear alcohol with 7 moles of ethylene oxide), Neodol™ 45-5 (the condensation product of C₁₄-C₁₅ linear alcohol with 5 moles of ethylene oxide) marketed by Shell Chemical Company, Kyro™ EOB (the condensation product of C₁₃-C₁₅ alcohol with 9 moles ethylene oxide), marketed by The Procter & Gamble Company, and Genapol LA 030 or O5O (the condensation product of C₁₂-C₁₄ alcohol with 3 or 5 moles of ethylene oxide) marketed by Hoechst. Preferred range of HLB in these products is from 8-11 and most preferred from 8-10.

Also useful as nonionic surfactants of the surfactant systems of the present invention are the alkylpolysaccharides disclosed in U.S. Patent 4,565,647, Lienado, issued January 21, 1986, having a hydrophobic group containing from 6 to 30 carbon atoms, preferably from 10 to 16 carbon atoms and a polysaccharide, e.g. a polyglycoside, hydrophilic group containing from 1.3 to 10, preferably from 1.3 to 3, most preferably from 1.3 to 2.7 saccharide units. Any reducing saccharide containing 5 or 6 carbon atoms can be used, e.g., glucose, galactose and galactosyl moieties can be substituted for the glucosyl moieties (optionally the hydrophobic group is attached at the 2-, 3-, 4-, etc. positions thus giving a glucose or galactose as opposed to a glucoside or galactoside). The intersaccharide bonds can be, e.g., between the one position of the additional saccharide units and the 2-, 3-, 4-, and/or 6- positions on the preceding saccharide units. The preferred alkylpolyglycosides have the formula

R²O(CₙH₂ₙO)ₜ(glycosyl)ₓ

wherein R² is selected from the group consisting of alkyl, alkylphenyl, hydroxyalkyl, hydroxyalkylphenyl, and mixtures thereof in which the alkyl groups contain from 10 to 18, preferably from 12 to 14, carbon atoms; n is 2 or 3, preferably 2; t is from 0 to 10, preferably 0; and x is from 1.3 to 10, preferably from 1.3 to 3, most preferably from 1.3 to 2.7. The glycosyl is preferably derived from glucose. To prepare these compounds, the alcohol or alkylpolyethoxy alcohol is formed first and then reacted with glucose, or a source of glucose, to form the glucoside (attachment at the 1-position). The additional glycosyl units can then be attached between their 1-position and the preceding glycosyl units 2-, 3-, 4- and/or 6-position, preferably predominately the 2-position.

The condensation products of ethylene oxide with a hydrophobic base formed by the condensation of propylene oxide with propylene glycol are also suitable for use as nonionic surfactants for the present invention. The hydrophobic portion of these compounds will preferably have a molecular weight of from 1500 to 1800 and will exhibit water insolubility. The addition of polyoxyethylene moieties to this hydrophobic portion tends to increase the water solubility of the molecule as a whole, and the liquid character of the product is retained up to the point where the polyoxyethylene content is 50% of the total weight of the condensation product, which corresponds to condensation with up to about 40 moles of ethylene oxide. Examples of compounds of this type include certain of the commercially-available Plurafac™ LF404 and Pluronic™ surfactants, marketed by BASF.

Further suitable nonionic surfactants for the purposes of the present invention, are the condensation products of ethylene oxide with the product resulting from the reaction of propylene oxide and ethylenediamine. The hydrophobic moiety of these products consists of the reaction product of ethylenediamine and excess propylene oxide, and generally has a molecular weight of from 2500 to 3000. This hydrophobic moiety is condensed with ethylene oxide to the extent that the condensation product contains from 40% to 80% by weight of polyoxyethylene and has a molecular weight of from 5,000 to 11,000. Examples of this type of nonionic surfactant include certain of the commercially available Tetronic™ compounds, marketed by BASF.

Preferred for use as nonionic surfactants of the surfactant systems of the present invention are polyethylene oxide condensates of alkyl phenols, condensation products of primary and secondary aliphatic alcohols with from 1 to 25 moles of ethylene oxide, alkylpolysaccharides, and mixtures thereof. Most preferred are C₈-C₁₄ alkyl phenol ethoxylates having from 3 to 15 ethoxy groups and C₈-C₁₈ alcohol ethoxylates (preferably C₁₀ avg.) having from 2 to 10 ethoxy groups, and mixtures thereof.

Highly preferred nonionic surfactants are polyhydroxy fatty acid amide surfactants of the formula : 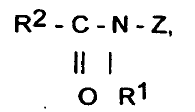 wherein R¹ is H, or R¹ is C₁₋₄ hydrocarbyl, 2-hydroxy ethyl, 2-hydroxy propyl or a mixture thereof, R² is C₅₋₃₁ hydrocarbyl, and Z is a polyhydroxyhydrocarbyl having a linear hydrocarbyl chain with at least 3 hydroxyls directly connected to the chain, or an alkoxylated derivative thereof. Preferably, R¹ is methyl, R² is a straight C₁₁₋₁₅ alkyl or C₁₆₋₁₈ alkyl or alkenyl chain such as coconut alkyl or mixtures thereof, and Z is derived from a reducing sugar such as glucose; fructose, maltose, lactose, in a reductive amination reaction.

Anionic surfactants : Suitable anionic surfactants for use in the surfactant systems of the present invention, are linear alkyl benzene sulfonate, alkyl ester sulfonate surfactants including linear esters of C₈-C₂₀ carboxylic acids (i.e., fatty acids) which are sulfonated with gaseous SO₃ according to "The Journal of the American Oil Chemists Society", 52 (1975), pp. 323-329. Suitable starting materials would include natural fatty substances as derived from tallow, palm oil, etc.
The preferred alkyl ester sulfonate surfactant, especially for laundry applications, comprise alkyl ester sulfonate surfactants of the structural formula: 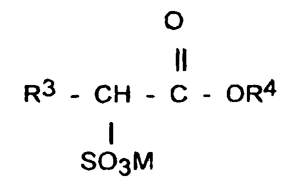 wherein R³ is a C₈-C₂₀ hydrocarbyl, preferably an alkyl, or combination thereof, R⁴ is a C₁-C₆ hydrocarbyl, preferably an alkyl, or combination thereof, and M is a cation which forms a water soluble salt with the alkyl ester sulfonate. Suitable salt-forming cations include metals such as sodium, potassium, and lithium, and substituted or unsubstituted ammonium cations, such as mono-ethanolamine, diethanolamine, and triethanolamine. Preferably, R³ is C₁₀-C₁₆ alkyl, and R⁴ is methyl, ethyl or isopropyl. Especially preferred are the methyl ester sulfonates wherein R³ is C₁₀-C₁₆ alkyl.

Other suitable anionic surfactants suitable for the surfactant systems herein, include the alkyl sulfate surfactants which are water soluble salts or acids of the formula ROSO₃M wherein R preferably is a C₁₀-C₂₄ hydrocarbyl, preferably an alkyl or hydroxyalkyl having a C₁₀-C₂₀ alkyl component, more preferably a C₁₂₋C₁₈ alkyl or hydroxyalkyl, and M is H or a cation, e.g., an alkali metal cation (e.g. sodium, potassium, lithium), or ammonium or substituted ammonium (e.g.. methyl-, dimethyl-, and trimethyl ammonium cations and quaternary ammonium cations such as tetramethyl-ammonium and dimethyl piperdinium cations and quaternary ammonium cations derived from alkylamines such as ethylamine, diethylamine, triethylamine, and mixtures thereof, and the like). Typically, alkyl chains of C₁₂-C₁₆ are preferred for lower wash temperatures (e.g. below about 50°C) and C₁₆₋₁₈ alkyl chains are preferred for higher wash temperatures (e.g. above 50°C).

Other anionic surfactants suitable for use in the surfactant systems of the present invention, include salts (including, for example, sodium, potassium, ammonium, and substituted ammonium salts such as mono-, di- and triethanolamine salts) of soap, C₈-C₂₂ primary of secondary alkanesulfonates, C₈-C₂₄ olefinsulfonates, sulfonated polycarboxylic acids prepared by sulfonation of the pyrolyzed product of alkaline earth metal citrates, e.g., as described in British patent specification No. 1,082,179, C₈-C₂₄ alkylpolyglycolethersulfates (containing up to 10 moles of ethylene oxide); alkyl glycerol sulfonates, fatty acyl glycerol sulfonates, fatty oleyl glycerol sulfates, alkyl phenol ethylene oxide ether sulfates, paraffin sulfonates, alkyl phosphates, isethionates such as the acyl isethionates, N-acyl taurates, alkyl succinamates and sulfosuccinates, monoesters of sulfosuccinates (especially saturated and unsaturated C₁₂-C₁₈ monoesters) and diesters of sulfosuccinates (especially saturated and unsaturated C₆-C₁₂ diesters), acyl sarcosinates, sulfates of alkylpolysaccharides such as the sulfates of alkylpolyglucoside (the nonionic nonsulfated compounds being described below), branched primary alkyl sulfates, and alkyl polyethoxy carboxylates such as those of the formula RO(CH₂CH₂O)ₖ-CH₂COO-M+ wherein R is a C₈-C₂₂ alkyl, k is an integer from 1 to 10, and M is a soluble salt-forming cation. Resin acids and hydrogenated resin acids are also suitable, such as rosin, hydrogenated rosin, and resin acids and hydrogenated resin acids present in or derived from tall oil. Further examples are described in "Surface Active Agents and Detergents" (Vol. I and II by Schwartz, Perry and Berch). A variety of such surfactants are also generally disclosed in U.S. Patent 3,929,678, issued December 30, 1975 to Laughlin, et al. at Column 23, line 58 through Column 29, line 23.
When included therein, the laundry detergent compositions of the present invention typically comprise from 1% to 40%, preferably from 3% to 20% by weight of such anionic surfactants.

Highly preferred anionic surfactants include alkyl alkoxylated sulfate surfactants hereof are water soluble salts or acids of the formula RO(A)ₘSO3M wherein R is an unsubstituted C₁₀-C₂₄ alkyl or hydroxyalkyl group having a C₁₀-C₂₄ alkyl component, preferably a C₁₂-C₂₀ alkyl or hydroxyalkyl, more preferably C₁₂₋C₁₈ alkyl or hydroxyalkyl, A is an ethoxy or propoxy unit, m is greater than zero, typically between 0.5 and 6, more preferably between 0.5 and about 3, and M is H or a cation which can be, for example, a metal cation (e.g., sodium, potassium, lithium, calcium, magnesium, etc.), ammonium or substituted-ammonium cation. Alkyl ethoxylated sulfates as well as alkyl propoxylated sulfates are contemplated herein. Specific examples of substituted ammonium cations include methyl-, dimethyl, trimethyl-ammonium cations and quaternary ammonium cations such as tetramethyl-ammonium and dimethyl piperdinium cations and those derived from alkylamines such as ethylamine, diethylamine, triethylamine, mixtures thereof, and the like. Exemplary surfactants are C₁₂-C₁₈ alkyl polyethoxylate (1.0) sulfate (C₁₂-C₁₈E(1.0)M), C₁₂-C₁₈ alkyl polyethoxylate (2.25) sulfate (C₁₂-C₁₈E(2.25)M), C₁₂-C₁₈ alkyl polyethoxylate (3.0) sulfate (C₁₂-C₁₈E(3.0)M), and C₁₂-C₁₈ alkyl polyethoxylate (4.0) sulfate (C₁₂-C₁₈E(4.0)M), wherein M is conveniently selected from sodium and potassium.

Further highly preferred anionic surfactants are the mid-chain-branched anionic surfactants described in the WO 97/38957, WO 97/39090, WO 97/38956, WO 97/39091, WO 97/38972, and in WO97/39091. These longer alkyl chain, mid-chain branched surfactant compounds are of the formula: A^{b} - X - B wherein:
(I) A^{b} is a hydrophobic mid-chain branched alkyl moiety, having in total 9 to 22 carbons in the moiety, preferably from 12 to 18, having: (1) a longest linear carbon chain attached to the - X - B moiety in the range of from 8 to 21 carbon atoms; (2) one or more C₁ - C₃ alkyl moieties branching from this longest linear carbon chain; (3) at least one of the branching alkyl moieties is attached directly to a carbon of the longest linear carbon chain at a position within the range of the position 2 carbon, counting from position 1 carbon (#1) which is attached to the - X - B moiety, to the position of the terminal carbon minus 2 carbons, (the (ω - 2) carbon); and (4) when more than one of these compounds is present, the average total number of carbon atoms in the A^{b}-X moieties in the above formula is within the range of greater than 11 to 20, preferably 14.5 to 18, more preferably from 15 to 17;
(II) B is a hydrophilic moiety selected from sulfates, sulfonates, amine oxides, polyoxyalkylene, preferably polyoxyethylene and polyoxypropylene, alkoxylated sulfates, polyhydroxy moieties, phosphate esters, glycerol sulfonates, polygluconates, polyphosphate esters, phosphonates, sulfosuccinates, sulfosuccaminates, polyalkoxylated carboxylates, glucamides, taurinates, sarcosinates, glycinates, isethionates, dialkanolamides, monoalkanolamides, monoalkanolamide sulfates, diglycolamides, diglycolamide sulfates, glycerol esters, glycerol ester sulfates, glycerol ethers, glycerol ether sulfates, polyglycerol ethers, polyglycerol ether sulfates, sorbitan esters, polyalkoxylated sorbitan esters, ammonioalkanesulfonates, amidopropyl betaines, alkylated quats, alkyated/polyhydroxyalkylated quats, alkylated quats, alkylated/polyhydroxylated oxypropyl quats, imidazolines, 2-yl-succinates, sulfonated alkyl esters, and sulfonated fatty acids; and
(III) X is selected from -CH₂- and -C(O)-.

Preferred surfactant systems herein comprise longer alkyl chain, mid-chain branched surfactant compounds of the above formula wherein the A^{b} moiety is a branched primary alkyl moiety having the formula: 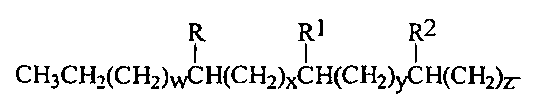 wherein the total number of carbon atoms in the branched primary alkyl moiety of this formula (including the R, R¹, and R² branching) is from 13 to 19; R, R¹, and R² are each independently selected from hydrogen and C₁-C₃ alkyl (preferably methyl), provided R, R¹, and R² are not all hydrogen and, when z is 0, at least R or R¹ is not hydrogen; w is an integer from 0 to 13; x is an integer from 0 to 13; y is an integer from 0 to 13; z is an integer from 0 to 13; and w + x + y + z is from 7 to 13.

In general, for the mid-chain branched surfactant compounds of the surfactant system, certain points of branching (e.g., the location along the chain of the R, R¹, and/or R² moieties in the above formula) are preferred over other points of branching along the backbone of the surfactant. The formula below illustrates the mid-chain branching range (i.e., where points of branching occur), preferred mid-chain branching range, and more preferred mid-chain branching range for mono-methyl branched alkyl A^{b} moieties useful according to the present invention. 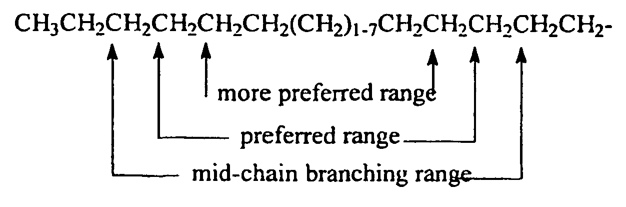 It should be noted that for the mono-methyl substituted surfactants these ranges exclude the two terminal carbon atoms of the chain and the carbon atom immediately adjacent to the -X - B group.

The formula below illustrates the mid-chain branching range, preferred mid-chain branching range, and more preferred mid-chain branching range for di-methyl substituted alkyl A^{b} moieties useful according to the present invention. 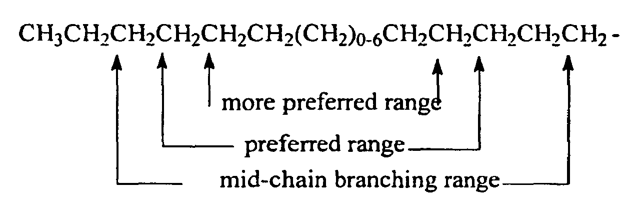 Preferred are surfactant compounds wherein in the above formula the A^{b} moiety does not have any quaternary substituted carbon atoms (i.e., 4 carbon atoms directly attached to one carbon atom).

The most preferred mid-chain branched surfactants compounds for use in the detergent compositions herein are mid-chain branched primary alkyl sulfonate and, even more preferably, sulfate surfactants. It should be understood that for the purpose of the invention, it may be preferred that the surfactant system comprises a mixture of two or more mid-chain branched primary alkyl sulfate or sulphonate surfactants.

Preferred mid-chain branched primary alkylsulfate surfactants are of the formula

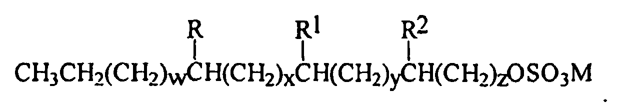

These surfactants have a linear primary alkyl sulfate chain backbone (i.e., the longest linear carbon chain which includes the sulfated carbon atom) which preferably comprises from 12 to 19 carbon atoms and their branched primary alkyl moieties comprise preferably a total of at least 14 and preferably no more than 20, carbon atoms. In the surfactant system comprising more than one of these sulfate surfactants, the average total number of carbon atoms for the branched primary alkyl moieties is preferably within the range of from greater than 11 to 20, preferably 14.5 to 17.5. Thus, the surfactant system preferably comprises at least one branched primary alkyl sulfate surfactant compound having a longest linear carbon chain of not less than 12 carbon atoms or not more than 19 carbon atoms, and the total number of carbon atoms including branching must be at least 14, and further the average total number of carbon atoms for the branched primary alkyl moiety is within the range of greater than 11 to 20, preferably 14.5 to 17.5.

R, R¹, and R² are each independently selected from hydrogen and C₁-C₃ alkyl group (preferably hydrogen or C₁-C₂ alkyl, more preferably hydrogen or methyl, and most preferably methyl), provided R, R¹, and R² are not all hydrogen. Further, when z is 1, at least R or R¹ is not hydrogen.

M is hydrogen or a salt forming cation depending upon the method of synthesis. Examples of salt forming cations are lithium, sodium, potassium, calcium, magnesium, quaternary alkyl amines having the formula 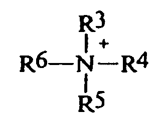 wherein R³, R⁴, R⁵ and R⁶ are independently hydrogen, C₁-C₂₂ alkylene, C₄₋C₂₂ branched alkylene, C₁-C₆ alkanol, C₁-C₂₂ alkenylene, C₄-C₂₂ branched alkenylene, and mixtures thereof. Preferred cations are ammonium (R³, R⁴, R⁵ and R⁶ equal hydrogen), sodium, potassium, mono-, di-, and trialkanol ammonium, and mixtures thereof. The monoalkanol ammonium compounds of the present invention have R³ equal to C₁-C₆ alkanol, R⁴, R⁵ and R⁶ equal to hydrogen; dialkanol ammonium compounds of the present invention have R³ and R⁴ equal to C₁-C₆ alkanol, R⁵ and R⁶ equal to hydrogen; trialkanol ammonium compounds of the present invention have R³, R⁴ and R⁵ equal to C₁-C₆ alkanol, R⁶ equal to hydrogen. Preferred alkanol ammonium salts of the present invention are the mono-, di- and tri- quaternary ammonium compounds having the formulas:

H₃N⁺CH₂CH₂OH, H₂N⁺(CH₂CH₂OH)₂, HN⁺(CH₂CH₂OH)₃.

Preferred M is sodium, potassium and the C₂ alkanol ammonium salts listed above; most preferred is sodium.

Further regarding the above formula, w is an integer from 0 to 13; x is an integer from 0 to 13; y is an integer from 0 to 13; z is an integer of at least 1; and w + x + y + z is an integer from 8 to 14.

A preferred mid-chain branched primary alkyl sulfate surfactant is, a C16 total carbon primary alkyl sulfate surfactant having 13 carbon atoms in the backbone and having 1, 2, or 3 branching units (i.e., R, R¹ and/or R²) of in total 3 carbon atoms, (whereby thus the total number of carbon atoms is at least 16). Preferred branching units can be one propyl branching unit or three methyl branching units.

Another preferred surfactant system of the present invention have one or more branched primary alkyl sulfates having the formula 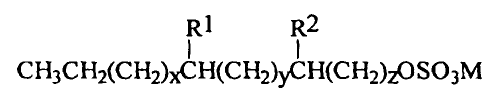 wherein the total number of carbon atoms, including branching, is from 15 to 18, and when more than one of these sulfates is present, the average total number of carbon atoms in the branched primary alkyl moieties having the above formula is within the range of greater than 11 to 20, preferably 14.5 to 17.5; R¹ and R² are each independently hydrogen or C₁-C₃ alkyl; M is a water soluble cation; x is from 0 to 11; y is from 0 to 11; z is at least 2; and x + y + z is from 9 to 13; provided R¹ and R² are not both hydrogen.

Preferably, the surfactant system comprises at least 20% by weight of the system, more preferably at least 60% by weight , even more preferably at least 90% by weight of the system, of a mid chain branched primary alkyl sulfates, preferably having R¹ and R² independently hydrogen or methyl, provided R¹ and R² are not both hydrogen; x + y is equal to 8, 9, or 10 and z is at least 2, whereby the average total number of carbon atoms in these sulfate surfactants is preferably from 15 to 17, more preferably from 16-17.

Furthermore, preferred surfactant systems are those, which comprise at least 20%, more preferably at least 60%, even more preferably at least 90% by weight of the system, of one or more mid-chain branched alkyl sulfates having the formula: 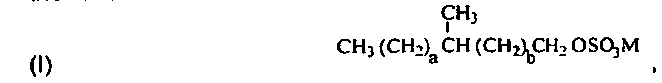 or 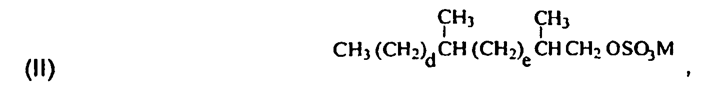 or mixtures thereof; wherein M represents one or more cations; a, b, d, and e are integers, a+b is from 10 to 16, d+e is from 8 to 14 and wherein further
when a + b = 10, a is an integer from 2 to 9 and b is an integer from 1 to 8;
when a + b = 11, a is an integer from 2 to 10 and b is an integer from 1 to 9;
when a + b = 12, a is an integer from 2 to 11 and b is an integer from 1 to 10;
when a + b = 13, a is an integer from 2 to 12 and b is an integer from 1 to 11;
when a + b = 14, a is an integer from 2 to 13 and b is an integer from 1 to 12;
when a + b = 15, a is an integer from 2 to 14 and b is an integer from 1 to 13;
when a + b = 16, a is an integer from 2 to 15 and b is an integer from 1 to 14;
when d + e = 8, d is an integer from 2 to 7 and e is an integer from 1 to 6;
when d + e = 9, d is an integer from 2 to 8 and e is an integer from 1 to 7;
when d + e = 10, d is an integer from 2 to 9 and e is an integer from 1 to 8;
when d + e = 11, d is an integer from 2 to 10 and e is an integer from 1 to 9;
when d + e = 12, d is an integer from 2 to 11 and e is an integer from 1 to 10;
when d + e = 13, d is an integer from 2 to 12 and e is an integer from 1 to 11;
when d + e = 14, d is an integer from 2 to 13 and e is an integer from 1 to 12;
whereby, when more than one of these sulfate surfactants is present in the surfactant system, the average total number of carbon atoms in the branched primary alkyl moieties having the above formulas is within the range of greater than 11 to 20, preferably 14.5 to about 17.5.

Preferred mono-methyl branched primary alkyl sulfates are selected from the group consisting of: 3-methyl pentadecanol sulfate, 4-methyl pentadecanol sulfate, 5-methyl pentadecanol sulfate, 6-methyl pentadecanol sulfate, 7-methyl pentadecanol sulfate, 8-methyl pentadecanol sulfate, 9-methyl pentadecanol sulfate, 10-methyl pentadecanol sulfate, 11-methyl pentadecanol sulfate, 12-methyl pentadecanol sulfate, 13-methyl pentadecanol sulfate, 3-methyl hexadecanol sulfate, 4-methyl hexadecanol sulfate, 5-methyl hexadecanol sulfate, 6-methyl hexadecanol sulfate, 7-methyl hexadecanol sulfate, 8-methyl hexadecanol sulfate, 9-methyl hexadecanol sulfate, 10-methyl hexadecanol sulfate, 11-methyl hexadecanol sulfate, 12-methyl hexadecanol sulfate, 13-methyl hexadecanol sulfate, 14-methyl hexadecanol sulfate, and mixtures thereof.

Preferred di-methyl branched primary alkyl sulfates are selected from the group consisting of: 2,3-methyl tetradecanol sulfate, 2,4-methyl tetradecanol sulfate, 2,5-methyl tetradecanol sulfate, 2,6-methyl tetradecanol sulfate, 2,7-methyl tetradecanol sulfate, 2,8-methyl tetradecanol sulfate, 2,9-methyl tetradecanol sulfate, 2,10-methyl tetradecanol sulfate, 2,11-methyl tetradecanol sulfate, 2,12-methyl tetradecanol sulfate, 2,3-methyl pentadecanol sulfate, 2,4-methyl pentadecanol sulfate, 2,5-methyl pentadecanol sulfate, 2,6-methyl pentadecanol sulfate, 2,7-methyl pentadecanol sulfate, 2,8-methyl pentadecanol sulfate, 2,9-methyl pentadecanol sulfate, 2,10-methyl pentadecanol sulfate, 2,11-methyl pentadecanol sulfate, 2,12-methyl pentadecanol sulfate, 2,13-methyl pentadecanol sulfate, and mixtures thereof.

The following branched primary alkyl sulfates comprising 16 carbon atoms and having one branching unit are examples of preferred branched surfactants useful in the present invention compositions:
5-methylpentadecylsulfate having the formula: 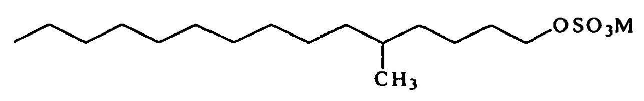
6-methylpentadecylsulfate having the formula 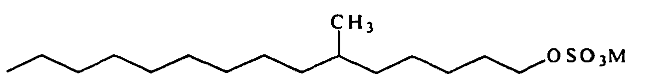
7-methylpentadecylsulfate having the formula 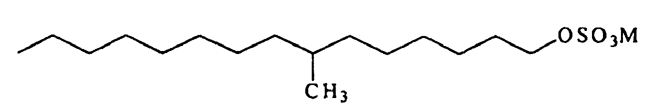
8-methylpentadecylsulfate having the formula 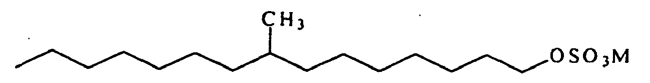
9-methylpentadecylsulfate having the formula 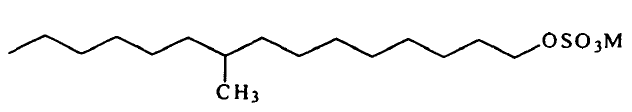
10-methylpentadecylsulfate having the formula 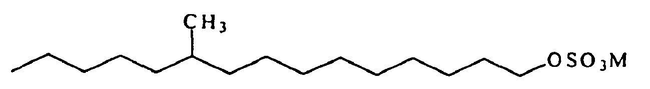 wherein M is preferably sodium.

The following branched primary alkyl sulfates comprising 17 carbon atoms and having two branching units are examples of preferred branched surfactants according to the present invention:
2,5-dimethylpentadecylsulfate having the formula: 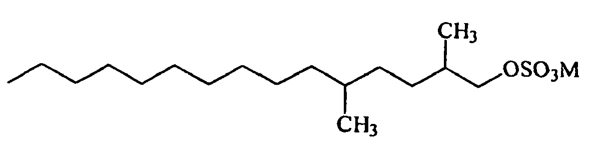
2,6-dimethylpentadecylsulfate having the formula 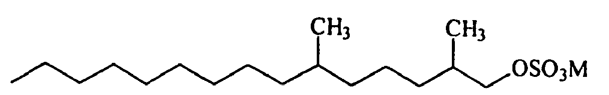
2,7-dimethylpentadecylsulfate having the formula 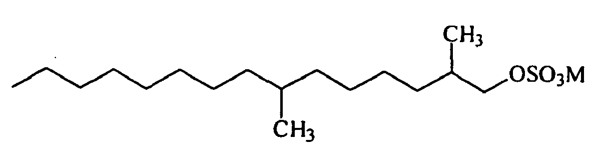
2,8-dimethylpentadecylsulfate having the formula 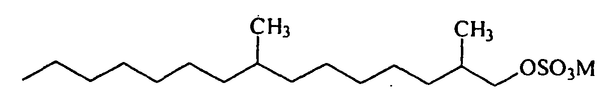
2,9-dimethylpentadecylsulfate having the formula 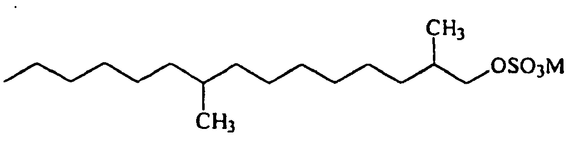
2,10-dimethylpentadecylsulfate having the formula 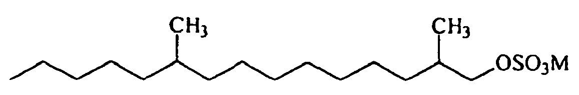 wherein M is preferably sodium.

The mid-chain branched encompasses in the present invention can be manufactured according to the process described in WO97/38956.

Cationic surfactant : Suitable cationic surfactants suitable for detersive purposes herein, are those having one long-chain hydrocarbyl group. Examples of such cationic surfactants include the ammonium surfactants such as alkyltrimethylammonium halogenides, and those surfactants having the formula

[R²(OR³)_{y}][R⁴(OR³)_{y}]₂R⁵N+X-

wherein R² is an alkyl or alkyl benzyl group having from 8 to 18 carbon atoms in the alkyl chain, each R³ is selected from the group consisting of -CH₂CH₂-, -CH₂CH(CH₃)-, -CH₂CH(CH₂OH)-, -CH₂CH₂CH₂-, and mixtures thereof; each R⁴ is selected from the group consisting of C₁-C₄ alkyl, C₁-C₄ hydroxyalkyl, benzyl ring structures formed by joining the two R⁴ groups, - CH₂CHOH-CHOHCOR⁶CHOHCH₂OH wherein R⁶ is any hexose or hexose polymer having a molecular weight less than 1000, and, hydrogen when y is not 0; R⁵ is the same as R⁴ or is an alkyl chain wherein the total number of carbon atoms of R² plus R⁵ is not more than 18; each y is from 0 to 10 and the sum of the y values is from 0 to 15; and X is any compatible anion.

Quaternary ammonium surfactant suitable for the present invention has the formula (I): 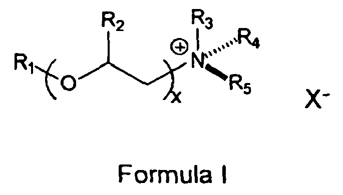 whereby R1 is a short chainlength alkyl (C6-C10) or alkylamidoalkyl of the formula (II) : 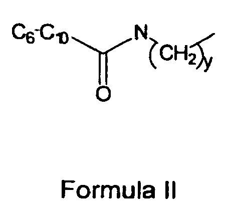 y is 2-4, preferably 3.
whereby R2 is H or a C1-C3 alkyl,
whereby x is 0-4, preferably 0-2, most preferably 0,
whereby R3, R4 and R5 are either the same or different and can be either a short chain alkyl (C1-C3) or alkoxylated alkyl of the formula III,
whereby X- is a counterion, preferably a halide, e.g. chloride or methylsulfate. 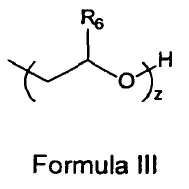 R6 is C₁-C₄ and z is 1 or 2.

Preferred quaternary ammonium surfactants are those as defined in formula I whereby
R₁ is C₈, C₁₀ or mixtures thereof, x=o,
R₃, R₄ = CH₃ and R₅ = CH₂CH₂OH.

Highly preferred cationic surfactants are the water-soluble quaternary ammonium compounds useful in the present composition having the formula :

R₁R₂R₃R₄N⁺X⁻ (i)

wherein R₁ is C₈-C₁₆ alkyl, each of R₂, R₃ and R₄ is independently C₁-C₄ alkyl, C₁-C₄ hydroxy alkyl, benzyl, and -(C₂H₄₀)ₓH where x has a value from 2 to 5, and X is an anion. Not more than one of R₂, R₃ or R₄ should be benzyl. The preferred alkyl chain length for R₁ is C₁₂-C₁₅ particularly where the alkyl group is a mixture of chain lengths derived from coconut or palm kernel fat or is derived synthetically by olefin build up or OXO alcohols synthesis. Preferred groups for R₂R₃ and R₄ are methyl and hydroxyethyl groups and the anion X may be selected from halide, methosulphate, acetate and phosphate ions.
Examples of suitable quaternary ammonium compounds of formulae (i) for use herein are :
coconut trimethyl ammonium chloride or bromide;
coconut methyl dihydroxyethyl ammonium chloride or bromide;
decyl triethyl ammonium chloride;
decyl dimethyl hydroxyethyl ammonium chloride or bromide;
C₁₂₋₁₅ dimethyl hydroxyethyl ammonium chloride or bromide;
coconut dimethyl hydroxyethyl ammonium chloride or bromide;
myristyl trimethyl ammonium methyl sulphate;
lauryl dimethyl benzyl ammonium chloride or bromide;
lauryl dimethyl (ethenoxy)₄ ammonium chloride or bromide;
choline esters (compounds of formula (i) wherein R₁ is 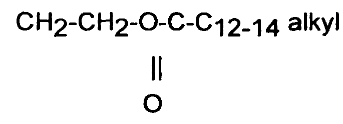 and R₂R₃R₄ are methyl).
di-alkyl imidazolines [compounds of formula (i)].

Other cationic surfactants useful herein are also described in U.S. Patent 4,228,044, Cambre, issued October 14, 1980 and in European Patent Application EP 000,224.

Typical fabric softening cationic surfactants include the water-insoluble quaternary-ammonium fabric softening actives or their corresponding amine precursor, the most commonly used having been di-long alkyl chain ammonium chloride or methyl sulfate.
Preferred cationic softeners among these include the following:
1) ditallow dimethylammonium chloride (DTDMAC);
2) dihydrogenated tallow dimethylammonium chloride;
3) dihydrogenated tallow dimethylammonium methylsulfate;
4) distearyl dimethylammonium chloride;
5) dioleyl dimethylammonium chloride;
6) dipalmityl hydroxyethyl methylammonium chloride;
7) stearyl benzyl dimethylammonium chloride;
8) tallow trimethylammonium chloride;
9) hydrogenated tallow trimethylammonium chloride;
10) C₁₂₋₁₄ alkyl hydroxyethyl dimethylammonium chloride;
11) C₁₂₋₁₈ alkyl dihydroxyethyl methylammonium chloride;
12) di(stearoyloxyethyl) dimethylammonium chloride (DSOEDMAC);
13) di(tallow-oxy-ethyl) dimethylammonium chloride;
14) ditallow imidazolinium methylsulfate;
15) 1-(2-tallowylamidoethyl)-2-tallowyl imidazolinium methylsulfate.

Biodegradable quaternary ammonium compounds have been presented as alternatives to the traditionally used di-long alkyl chain ammonium chlorides and methyl sulfates. Such quaternary ammonium compounds contain long chain alk(en)yl groups interrupted by functional groups such as carboxy groups. Said materials and fabric softening compositions containing them are disclosed in numerous publications such as EP-A-0,040,562, and EP-A-0,239,910.

The quaternary ammonium compounds and amine precursors herein have the formula (I) or (II), below : 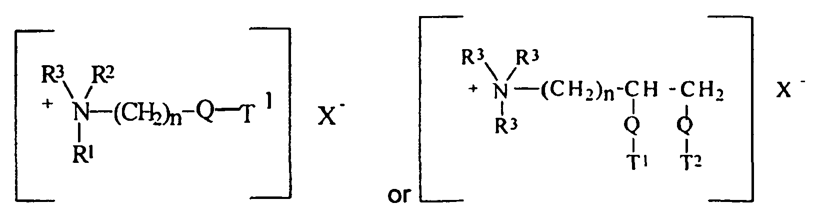 wherein Q is selected from -O-C(O)-, -C(O)-O-, -O-C(O)-O-, -NR⁴-C(O)-, -C(O)-NR⁴⁻;
R¹ is (CH₂)ₙ-Q-T² or T³;
R² is (CH₂)ₘ-Q-T⁴ or T⁵ or R³;
R³ is C₁-C₄ alkyl or C₁-C₄ hydroxyalkyl or H;
R⁴ is H or C₁-C₄ alkyl or C₁-C₄ hydroxyalkyl;
T¹, T², T³, T⁴, T⁵ are independently C₁₁-C₂₂ alkyl or alkenyl;
n and m are integers from 1 to 4; and
X⁻ is a softener-compatible anion.

Non-limiting examples of softener-compatible anions include chloride or methyl sulfate.

The alkyl, or alkenyl, chain T¹, T², T³, T⁴, T⁵ must contain at least 11 carbon atoms, preferably at least 16 carbon atoms. The chain may be straight or branched.
Tallow is a convenient and inexpensive source of long chain alkyl and alkenyl material. The compounds wherein T¹, T², T³, T⁴, T⁵ represents the mixture of long chain materials typical for tallow are particularly preferred.
Specific examples of quaternary ammonium compounds suitable for use in the aqueous fabric softening compositions herein include:
1) N,N-di(tallowyl-oxy-ethyl)-N,N-dimethyl ammonium chloride;
2) N,N-di(tallowyl-oxy-ethyl)-N-methyl, N-(2-hydroxyethyl) ammonium methyl sulfate;
3) N,N-di(2-taliowyi-oxy-2-oxo-ethyl)-N,N-dimethyl ammonium chloride;
4) N,N-di(2-tallowyl-oxy-ethylcarbonyl-oxy-ethyl)-N,N-dimethyl ammonium chloride;
5) N-(2-tallowyl-oxy-2-ethyl)-N-(2-tallowyl-oxy-2-oxo-ethyl)-N,N-dimethyl ammonium chloride;
6) N,N,N-tri(tallowyl-oxy-ethyl)-N-methyl ammonium chloride;
7) N-(2-tallowyl-oxy-2-oxo-ethyl)-N-(tallowyl-N,N-dimethyl-ammonium chloride; and
8) 1,2-ditallowyl-oxy-3-trimethylammoniopropane chloride;
and mixtures of any of the above materials.

The surfactant is preferably formulated to be compatible with enzyme components present in the composition. In liquid or gel compositions, the surfactant is most preferably formulated such that it promotes, or at least does not degrade, the stability of any enzyme in these compositions. The compositions of the present invention can further comprise ampholytic, zwitterionic and or semi-polar surfactants.

Ampholytic surfactants are suitable for use in the laundry detergent and/or fabric care compositions of the present invention can be broadly described as aliphatic derivatives of secondary or tertiary amines, or aliphatic derivatives of heterocyclic secondary and tertiary amines in which the aliphatic radical can be straight- or branched-chain. One of the aliphatic substituents contains at least 8 carbon atoms, typically from 8 to 18 carbon atoms, and at least one contains an anionic water-solubilizing group, e.g. carboxy, sulfonate, sulfate. See U.S. Patent No. 3,929,678 to Laughlin et al., issued December 30, 1975 at column 19, lines 18-35, for examples of ampholytic surfactants. When included therein, the laundry detergent and/or fabric care compositions of the present invention typically comprise from 0.2% to 15%, preferably from 1% to 10% by weight of such ampholytic surfactants.

Zwitterionic surfactants suitable for use in the laundry detergent and/or fabric care compositions can be broadly described as derivatives of secondary and tertiary amines, derivatives of heterocyclic secondary and tertiary amines, or derivatives of quaternary ammonium, quaternary phosphonium or tertiary sulfonium compounds. See U.S. Patent No. 3,929,678 to Laughlin et al., issued December 30, 1975 at column 19, line 38 through column 22, line 48, for examples of zwitterionic surfactants.
When included therein, the laundry detergent and/or fabric care compositions of the present invention typically comprise from 0.2% to 15%, preferably from 1 % to 10% by weight of such zwitterionic surfactants.

Semi-polar nonionic surfactants are a special category of nonionic surfactants which include water-soluble amine oxides containing one alkyl moiety of from 10 to 18 carbon atoms and 2 moieties selected from the group consisting of alkyl groups and hydroxyalkyl groups containing from 1 to 3 carbon atoms; water-soluble phosphine oxides containing one alkyl moiety of from 10 to 18 carbon atoms and 2 moieties selected from the group consisting of alkyl groups and hydroxyalkyl groups containing from 1 to 3 carbon atoms; and water-soluble sulfoxides containing one alkyl moiety of from 10 to 18 carbon atoms and a moiety selected from the group consisting of alkyl and hydroxyalkyl moieties of from 1 to 3 carbon atoms.
Semi-polar nonionic detergent surfactants include the amine oxide surfactants having the formula 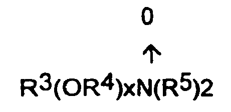 wherein R³ is an alkyl, hydroxyalkyl, or alkyl phenyl group or mixtures therof containing from 8 to 22 carbon atoms; R⁴ is an alkylene or hydroxyalkylene group containing from 2 to 3 carbon atoms or mixtures thereof; x is from 0 to 3; and each R⁵ is an alkyl or hydroxyalkyl group containing from 1 to 3 carbon atoms or a polyethylene oxide group containing from 1 to 3 ethylene oxide groups. The R⁵ groups can be attached to each other, e.g., through an oxygen or nitrogen atom, to form a ring structure.
These amine oxide surfactants in particular include C₁₀-C₁₈ alkyl dimethyl amine oxides and C₈-C₁₂ alkoxy ethyl dihydroxy ethyl amine oxides.
When included therein, the compositions of the present invention typically comprise from 0.2% to 15%, preferably from 1% to 10% by weight of such semi-polar nonionic surfactants.

Surfactants selected from the group of primary or tertiary amines also suitable for use in the laundry detergent and/or fabric care compositions of the present invention.
Suitable primary amines for use herein include amines according to the formula R₁NH₂ wherein R₁ is a C₆-C₁₂, preferably C₆-C₁₀ alkyl chain or R₄X(CH₂)ₙ, X is -O-,-C(O)NH- or -NH- R₄ is a C₆-C₁₂ alkyl chain n is between 1 to 5, preferably 3. R₁ alkyl chains may be straight or branched and may be interrupted with up to 12, preferably less than 5 ethylene oxide moieties.
Preferred amines according to the formula herein above are n-alkyl amines. Suitable amines for use herein may be selected from 1-hexylamine, 1-octylamine, 1-decylamine and laurylamine. Other preferred primary amines include C8-C10 oxypropylamine, octyloxypropylamine, 2-ethylhexyloxypropylamine, lauryl amido propylamine and amido propylamine.
Suitable tertiary amines for use herein include tertiary amines having the formula R₁R₂R₃N wherein R1 and R2 are C₁-C₈ alkylchains or 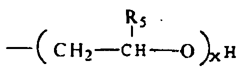 R₃ is either a C₆-C₁₂, preferably C₆-C₁₀ alkyl chain, or R₃ is R₄X(CH₂)ₙ, whereby X is -O-, -C(O)NH- or -NH-, R₄ is a C₄-C₁₂, n is between 1 to 5, preferably 2-3. R₅ is H or C₁-C₂ alkyl and x is between 1 to 6 .
R₃ and R₄ may be linear or branched ; R₃ alkyl chains may be interrupted with up to 12, preferably less than 5, ethylene oxide moieties.
Preferred tertiary amines are R₁R₂R₃N where R1 is a C6-C12 alkyl chain, R2 and R3 are C1-C3 alkyl or 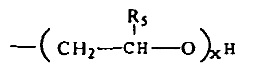 where R5 is H or CH3 and x = 1-2.
Also preferred are the amidoamines of the formula: 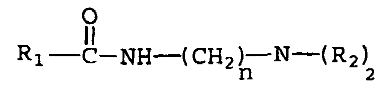 wherein R₁ is C₆-C₁₂ alkyl; n is 2-4,
preferably n is 3; R₂ and R₃ is C₁-C₄

Most preferred amines of the present invention include 1-octylamine, 1-hexylamine, 1-decylamine, 1-dodecylamine,C8-10oxypropylamine, N coco 1-3diaminopropane, coconutalkyldimethylamine, lauryldimethylamine, lauryl bis(hydroxyethyl)amine, coco bis(hydroxyehtyl)amine, lauryl amine 2 moles propoxylated, octyl amine 2 moles propoxylated, lauryl amidopropyldimethylamine, C8-10 amidopropyldimethylamine and C10 amidopropyldimethylamine.
The most preferred amines for use in the compositions herein are 1-hexylamine, 1-octylamine, 1-decylamine, 1-dodecylamine. Especially desirable are n-dodecyldimethylamine and bishydroxyethylcoconutalkylamine and oleylamine 7 times ethoxylated, lauryl amido propylamine and cocoamido propylamine.

### THE PROTEASE

The third essential element of the laundry detergent and/or fabric care compositions according to the present invention is a protelytic enzyme. It has been surprisingly found that the laundry detergent and/or fabric care compositions of the present invention comprising a chemical entity, a surfactant and a protease achieve superior and improved cleaning and/or fabric care performance. Without wishing to be bound by theory, it is believed that the binding of chemical entity to the fabric, disrupts the fabric fibers leading to the loosening of the soils and stains. This results in the protease having increased accessibility to laundry soils / stains and the fabric enabling the protease to efficiently remove such soils / stains. This further results in the surfatant and chemical entity to have increased accessibility to laundry soils / stains and to the fabric enhancing their performances.

Suitable proteases are the subtilisins which are obtained from particular strains of *B. subtilis* and *B. licheniformis* (subtilisin BPN and BPN'). One suitable protease is obtained from a strain of *Bacillus,* having maximum activity throughout the pH range of 8-12, developed and sold as ESPERASE® by Novo Industries A/S of Denmark, hereinafter "Novo". The preparation of this enzyme and analogous enzymes is described in GB 1,243,784 to Novo. Other suitable proteases include ALCALASE®, DURAZYM® and SAVINASE® from Novo and MAXATASE®, MAXACAL®, PROPERASE® and MAXAPEM® (protein engineered Maxacal) from Gist-Brocades. Also suitable for the present invention are proteases described in patent applications EP 251 446 and WO 91/06637, protease BLAP® described in WO91/02792 and their variants described in WO 95/23221. See also a high pH protease from Bacillus sp. NCIMB 40338 described in WO 93/18140 A to Novo. Enzymatic detergents comprising protease, one or more other enzymes, and a reversible protease inhibitor are described in WO 92/03529 A to Novo. When desired, a protease having decreased adsorption and increased hydrolysis is available as described in WO 95/07791 to Procter & Gamble. A recombinant trypsin-like protease for detergents suitable herein is described in WO 94/25583 to Novo. Other suitable proteases are described in EP 516 200 by Unilever.

Proteolytic enzymes also encompass modified bacterial serine proteases, such as those described in European Patent Application Serial Number 87 303761.8, filed April 28, 1987 (particularly pages 17, 24 and 98), and which is called herein "Protease B", and in European Patent Application 199,404, Venegas, published October 29, 1986, which refers to a modified bacterial serine protealytic enzyme which is called "Protease A" herein. Suitable is what is called herein "Protease C", which is a variant of an alkaline serine protease from *Bacillus* in which lysine replaced arginine at position 27, tyrosine replaced valine at position 104, serine replaced asparagine at position 123, and alanine replaced threonine at position 274. Protease C is described in WO 91/06637. Genetically modified variants, particularly of Protease C, are also included herein.

A preferred protease referred to as "Protease D" is a carbonyl hydrolase variant having an amino acid sequence not found in nature, which is derived from a precursor carbonyl hydrolase by substituting a different amino acid for a plurality of amino acid residues at a position in said carbonyl hydrolase equivalent to position +76, preferably also in combination with one or more amino acid residue positions equivalent to those selected from the group consisting of +99, +101, +103, +104, +107, +123, +27, +105, +109, +126, +128, +135, +156, +166, +195, +197, +204, +206, +210, +216, +217, +218, +222, +260, +265, and/or +274 according to the numbering of *Bacillus amyloliquefaciens* subtilisin, as described in WO95/1059 and in WO95/10592. The "protease D" variants have preferably the amino acid substitution set 76/103/104, more preferably the substitution set N76D/S103A/V1041. Also suitable is a carbonyl hydrolase variant of the protease described in WO95/10591. having an amino acid sequence derived by replacement of a plurality of amino acid residues replaced in the precursor enzyme corresponding to position +210 in combination with one or more of the following residues : +33, +62, +67, +76, +100, +101, +103, +104, +107, +128, +129, +130, +132, +135, +156, +158, +164, +166, +167, +170, +209, +215, +217, +218, and +222, where the numbered position corresponds to naturally-occurring subtilisin from *Bacillus amyloliquefaciens* or to equivalent amino acid residues in other carbonyl hydrolases or subtilisins, such as *Bacillus lentus* subtilisin (co-pending patent WO98/55634).

More preferred proteases are multiply-substituted protease variants. These protease variants comprise a substitution of an amino acid residue with another naturally occuring amino acid residue at an amino acid residue position corresponding to position 103 of *Bacillus amyloliquefaciens* subtilisin in combination with a substitution of an amino acid residue positions corresponding to positions 1, 3, 4, 8, 9, 10, 12, 13, 16, 17, 18, 19, 20, 21, 22, 24, 27, 33, 37, 38, 42, 43, 48, 55, 57, 58, 61, 62, 68, 72, 75, 76, 77, 78, 79, 86, 87, 89, 97, 98, 99, 101, 102, 104, 106, 107, 109, 111, 114, 116, 117, 119, 121, 123, 126, 128, 130, 131, 133, 134, 137, 140, 141, 142, 146, 147, 158, 159, 160, 166, 167, 170, 173, 174, 177, 181, 182, 183, 184, 185, 188, 192, 194, 198, 203, 204, 205, 206, 209, 210, 211, 212, 213, 214, 215, 216, 217, 218, 222, 224, 227, 228, 230, 232, 236, 237, 238, 240, 242, 243, 244, 245, 246, 247, 248, 249, 251, 252, 253, 254, 255, 256, 257, 258, 259, 260, 261, 262, 263, 265, 268, 269, 270, 271, 272, 274 and 275 of *Bacillus amyloliquefaciens* subtilisin; wherein when said protease variant includes a substitution of amino acid residues at positions corresponding to positions 103 and 76, there is also a substitution of an amino acid residue at one or more amino acid residue positions other than amino acid residue positions corresponding to positions 27, 99, 101, 104, 107, 109, 123, 128, 166, 204, 206, 210, 216, 217, 218, 222, 260, 265 or 274 of *Bacillus amyloliquefaciens* subtilisin and/or multiply-substituted protease variants comprising a substitution of an amino acid residue with another naturally occuring amino acid residue at one or more amino acid residue positions corresponding to positions 62, 212, 230, 232, 252 and 257 of *Bacillus amyloliquefaciens* subtilisin as described in PCT application WO 99/20727, WO 89/20726 and WO 99/20723 all filed on October 23, 1998 from The Procter & Gamble Company. Preferred multiply substituted protease variants have te amino acid substitution set 101/103/104/159/232/236/245/248/252, more preferably 101G/103A/1041/159D/232V/236H/245R/248D/252K according to the numbering of *Bacillus amyloliquiefaciens subtilisin.*

For the purposes of the present invention, the most preferred proteases are selected from : "Protease B" as described above; "Protease C" as described above; the "Protease D" as described above having the amino acid substitution set 76/103/104, more preferably N76D/S103A/V1041 according to the numbering of *Bacillus lentus subtilisin;* a multiply-substituted protease variant comprising the amino acid substitution set 101/103/104/159/232/236/245/248/252, more preferably 101G/103A/1041/159D/232V/236H/245R/248D/252K according to the numbering of *Bacillus amyloliquiefaciens subtilisin* as described above and/or mixtures thereof.

The proteolytic enzymes are generally incorporated in the laundry detergent and/or fabric care compositions of the present invention at a level of from 0.0001% to 2%, preferably from 0.001% to 0.2%, more preferably from 0.005% to 0.1 % pure enzyme by weight of the total composition.

### LAUNDRY DETERGENT AND FABRIC CARE COMPONENTS

The laundry detergent and/or fabric care compositions of the invention may also contain additional detergent and/or fabric care components. The precise nature of these additional components, and levels of incorporation thereof will depend on the physical form of the composition, and the nature of the cleaning operation for which it is to be used.

As already mentioned vide supra, all the benefit agents that may be linked to a deposition aid and/or linking region in accordance with the present invention, can also be incorporated into the laundry detergent and/or fabric care compositions of the present invention in their unmodified, conventional form.

The compositions of the invention may for example, be formulated as hand and machine laundry detergent compositions including laundry additive compositions and compositions suitable for use in the soaking and/or pretreatment of stained fabrics, rinse added fabric softener compositions. Pre-or post treatment of fabric include gel, spray and liquid fabric care compositions. A rinse cycle with or without the presence of softening agents is also contemplated. The compositions of the invention can also be used as detergent additive products in solid or liquid form. Such additive products are intended to supplement or boost the performance of conventional detergent compositions and can be added at any stage of the cleaning process.

The laundry detergent and/or fabric care compositions according to the invention can be liquid, paste, gels, bars, tablets, spray, foam, powder or granular forms. Granular compositions can also be in "compact" form, the liquid compositions can also be in a "concentrated" form. If needed the density of the laundry detergent compositions herein ranges from 400 to 1200 g/litre, preferably 600 to 950 g/litre of composition measured at 20°C.
The "compact" form of the compositions herein is best reflected by density and, in terms of composition, by the amount of inorganic filler salt; inorganic filler salts are conventional ingredients of detergent compositions in powder form; in conventional detergent compositions, the filler salts are present in substantial amounts, typically 17-35% by weight of the total composition. In the compact compositions, the filler salt is present in amounts not exceeding 15% of the total composition, preferably not exceeding 10%, most preferably not exceeding 5% by weight of the composition. The inorganic filler salts, such as meant in the present compositions are selected from the alkali and alkaline-earth-metal salts of sulphates and chlorides. A preferred filler salt is sodium sulphate. Liquid detergent compositions according to the present invention can also be in a "concentrated form", in such case, the liquid detergent compositions according the present invention will contain a lower amount of water, compared to conventional liquid detergents. Typically the water content of the concentrated liquid detergent is preferably less than 40%, more preferably less than 30%, most preferably less than 20% by weight of the detergent composition.

### Conventional detergent enzymes

Conventional detergent enzymes for use in the laundry detergent and/or fabric care compositions of the present invention include enzymes selected from cellulases, hemicellulases, peroxidases, gluco-amylases, amylases, xylanases, lipases, phospholipases, esterases, cutinases, pectinases, keratanases, reductases, oxidases, phenoloxidases, lipoxygenases, ligninases, pullulanases, tannases, pentosanases, β-glucanases, arabinosidases, hyaluronidase, chondroitinase, laccase or mixtures thereof.

The laundry detergent and/or fabric care compositions of the present invention will preferably further comprise an amylase. Without wishing to be bound by theory, it is believed that amylases further boost the cleaning performance of the compositions of the present invention, by enhancing the removal of carbohydrate/starch containing soils and stains, starch from textile finishing treatment.
Suitable are α and/or β amylases such as in WO94/02597, Novo Nordisk A/S published February 03, 1994, describes cleaning compositions which incorporate mutant amylases. See also WO95/10603, Novo Nordisk A/S, published April 20, 1995. Other amylases known for use in cleaning compositions include both α- and β-amylases. α-Amylases are known in the art and include those disclosed in US Pat. no. 5,003,257; EP 252,666; WO/91/00353; FR 2,676,456; EP 285,123; EP 525,610; EP 368,341; and British Patent specification no. 1,296,839 (Novo). Other suitable amylases are stability-enhanced amylases described in WO94/18314, published August 18, 1994 and WO96/05295, Genencor, published February 22, 1996 and amylase variants having additional modification in the immediate parent available from Novo Nordisk A/S, disclosed in WO 95/10603, published April 95. Also suitable are amylases described in EP 277 216, WO95/26397 and WO96/23873 (all by Novo Nordisk). Examples of commercial α-amylases products are Purafect Ox Am® from Genencor and Termamyl®, Ban® ,Fungamyl® and Duramyl®, all available from Novo Nordisk A/S Denmark. WO95/26397 describes other suitable amylases : α-amylases characterised by having a specific activity at least 25% higher than the specific activity of Termamyl® at a temperature range of 25°C to 55°C and at a pH value in the range of 8 to 10, measured by the Phadebas® α-amylase activity assay. Preferred are variants of the above enzymes, described in WO96/23873 (Novo Nordisk). Preferably, the variants are those demonstrating improved thermal stability, more preferably those wherein at least one amino acid residue equivalent to F180, R181, G182, T183, G184, or K185 has been deleted from the parent α-amylase. Particularly preferred are those variants having improved thermal stability which comprise the amino acid deletions R181 + G182 or T183 + G184. Other amylolytic enzymes with improved properties with respect to the activity level and the combination of thermal stability and a higher activity level are described in WO95/35382.
The amylolytic enzymes are generally incorporated in the laundry detergent and/or fabric care compositions of the present invention at a level of from 0.0001% to 2%, preferably from 0.00018% to 0.06%, more preferably from 0.00024% to 0.048% pure enzyme by weight of the total composition.

Preferably for use in the present invention is a combination of conventional applicable enzymes like protease, amylase, lipase, cutinase and/or cellulase in conjunction with one or more plant cell wall degrading enzymes.

The cellulases usable in the present invention include both bacterial or fungal cellulases. Preferably, they will have a pH optimum of between 5 and 12 and a specific activity above 50 CEVU/mg (Cellulose Viscosity Unit). Suitable cellulases are disclosed in U.S. Patent 4,435,307, Barbesgoard et al, J61078384 and WO96/02653 which discloses fungal cellulase produced respectively from Humicola insolens, Trichoderma, Thielavia and Sporotrichum. EP 739 982 describes cellulases isolated from novel Bacillus species. Suitable cellulases are also disclosed in GB-A-2.075.028; GB-A-2.095.275; DE-OS-2.247.832 and WO95/26398.
Examples of such cellulases are cellulases produced by a strain of Humicola insolens (Humicola grisea var. thermoidea), particularly the Humicola strain DSM 1800.
Other suitable cellulases are cellulases originated from Humicola insolens having a molecular weight of about 50KDa, an isoelectric point of 5.5 and containing 415 amino acids; and a ⁻43kD endoglucanase derived from Humicola insolens, DSM 1800, exhibiting cellulase activity; a preferred endoglucanase component has the amino acid sequence disclosed in PCT Patent Application No. WO 91/17243. Also suitable cellulases are the EGIII cellulases from Trichoderma longibrachiatum described in WO94/21801, Genencor, published September 29, 1994. Especially suitable cellulases are the cellulases having color care benefits. Examples of such cellulases are cellulases described in European patent application No. 91202879.2, filed November 6, 1991 (Novo). Carezyme and Celluzyme (Novo Nordisk A/S) are especially useful. See also WO91/17244 and WO91/21801. Other suitable cellulases for fabric care and/or cleaning properties are described in WO96/34092, WO96/17994 and WO95/24471.
Said cellulases are normally incorporated in the compositions of the present invention, at levels from 0.0001% to 2% of pure enzyme by weight of the composition.

Peroxidase enzymes are used in combination with oxygen sources, e.g. percarbonate, perborate, persulfate, hydrogen peroxide, etc and with a phenolic substrate as bleach enhancing molecule. They are used for "solution bleaching", i.e. to prevent transfer of dyes or pigments removed from substrates during wash operations to other substrates in the wash solution. Peroxidase enzymes are known in the art, and include, for example, horseradish peroxidase, ligninase and haloperoxidase such as chloro- and bromo-peroxidase. Peroxidase-containing detergent compositions are disclosed, for example, in PCT International Application WO 89/099813, WO89/09813 and in European Patent application EP No. 91202882.6, filed on November 6, 1991 and EP No. 96870013.8, filed February 20, 1996. Also suitable is the laccase enzyme.
Enhancers are generally comprised at a level of from 0.1% to 5% by weight of total composition. Preferred enhancers are substitued phenthiazine and phenoxasine 10-Phenothiazinepropionicacid (PPT), 10-ethylphenothiazine-4-carboxylic acid (EPC), 10-phenoxazinepropionic acid (POP) and 10-methylphenoxazine (described in WO 94/12621) and substitued syringates (C3-C5 substitued alkyl syringates) and phenols. Sodium percarbonate or perborate are preferred sources of hydrogen peroxide.
Said peroxidases are normally incorporated in the compositions herein, at levels from 0.0001% to 2% of pure enzyme by weight of the composition.
Enzymatic system may be used as bleaching agents : The hydrogen peroxide may also be present by adding an enzymatic system (i.e. an enzyme and a substrate therefore) which is capable of generating hydrogen peroxide at the beginning or during the washing and/or rinsing process. Such enzymatic systems are disclosed in EP 537 381 filed October 9; 1991.

Other preferred enzymes that can be included in the compositions of the present invention include lipases. Suitable lipase enzymes for detergent usage include those produced by microorganisms of the Pseudomonas group, such as Pseudomonas stutzeri ATCC 19.154, as disclosed in British Patent 1,372,034. Suitable lipases include those which show a positive immunological cross-reaction with the antibody of the lipase, produced by the microorganism *Pseudomonas fluorescent* IAM 1057. This lipase is available from Amano Pharmaceutical Co. Ltd., Nagoya, Japan, under the trade name Lipase P "Amano," hereinafter referred to as "Amano-P". Other suitable commercial lipases include Amano-CES, lipases ex *Chromobacter viscosum,* e.g. *Chromobacter viscosum var. lipolyticum* NRRLB 3673 from Toyo Jozo Co., Tagata, Japan; *Chromobacter viscosum* lipases from U.S. Biochemical Corp., U.S.A. and Disoynth Co., The Netherlands, and lipases ex *Pseudomonas gladioli.* Especially suitable lipases are lipases such as M1 Lipase^{R} and Lipomax^{R} (Gist-Brocades) and Lipolase^{R} and Lipolase Ultra^{R}(Novo) which have found to be very effective when used in combination with the compositions of the present invention. Also suitables are the lipolytic enzymes described in EP 258 068, WO 92/05249 and WO 95/22615 by Novo Nordisk and in WO 94/03578, WO 95/35381 and WO 96/00292 by Unilever.
Also suitable are cutinases [EC 3.1.1.50] which can be considered as a special kind of lipase, namely lipases which do not require interfacial activation. Addition of cutinases to detergent compositions have been described in e.g. WO-A-88/09367 (Genencor); WO 90/09446 (Plant Genetic System) and WO 94/14963 and WO 94/14964 (Unilever). The lipases and/or cutinases are normally incorporated in the compositions of the present invention, at levels from 0.0001% to 2% of pure enzyme by weight of the composition.

The above-mentioned enzymes may be of any suitable origin, such as vegetable, animal, bacterial, fungal and yeast origin. Origin can further be mesophilic or extremophilic (psychrophilic, psychrotrophic, thermophilic, barophilic, alkalophilic, acidophilic, halophilic, etc.). Purified or non-purified forms of these enzymes may be used. Also included by definition, are mutants of native enzymes. Mutants can be obtained e.g. by protein and/or genetic engineering, chemical and/or physical modifications of native enzymes. Common practice as well is the expression of the enzyme via host organisms in which the genetic material responsible for the production of the enzyme has been cloned.

Said enzymes are normally incorporated in the laundry detergent and/or fabric care compositions at levels from 0.0001% to 2% of pure enzyme by weight of the composition. The enzymes can be added as separate single ingredients (prills, granulates, stabilized liquids, etc. containing one enzyme) or as mixtures of two or more enzymes (e.g. cogranulates).

Other suitable detergent ingredients that can be added are enzyme oxidation scavengers which are described in Copending European Patent application EP 553 607 filed on January 31, 1992. Examples of such enzyme oxidation scavengers are ethoxylated tetraethylene polyamines.

A range of enzyme materials and means for their incorporation into synthetic detergent compositions is also disclosed in WO 9307263 A and WO 9307260 A to Genencor International, WO 8908694 A to Novo, and U.S. 3,553,139, January 5, 1971 to McCarty et al. Enzymes are further disclosed in U.S. 4.101.457, Place et al, July 18, 1978, and in U.S. 4,507,219, Hughes, March 26, 1985. Enzyme materials useful for liquid detergent formulations, and their incorporation into such formulations, are disclosed in U.S. 4,261,868, Hora et al, April 14, 1981. Enzymes for use in detergents can be stabilised by various techniques. Enzyme stabilisation techniques are disclosed and exemplified in U.S. 3,600,319, August 17, 1971, Gedge et al, EP 199,405 and EP 200,586, October 29, 1986, Venegas. Enzyme stabilisation systems are also described, for example, in U.S. 3,519,570. A useful Bacillus, sp. AC13 giving proteases, xylanases and cellulases, is described in WO 9401532 A to Novo.

### Builder system

The laundry detergent and/or fabric care compositions according to the present invention may further comprise a builder system.

Any conventional builder system is suitable for use herein including aluminosilicate materials, silicates, polycarboxylates; alkyl- or alkenyl-succinic acid and fatty acids, materials such as ethylenediamine tetraacetate, diethylene triamine pentamethyleneacetate, metal ion sequestrants such as aminopolyphosphonates, particularly ethylenediamine tetramethylene phosphonic acid and diethylene triamine pentamethylenephosphonic acid. Phosphate builders can also be used herein. Suitable builders can be an inorganic ion exchange material, commonly an inorganic hydrated aluminosilicate material, more particularly a hydrated synthetic zeolite such as hydrated zeolite A, X, B, HS or MAP.
Another suitable inorganic builder material is layered silicate, e.g. SKS-6 (Hoechst). SKS-6 is a crystalline layered silicate consisting of sodium silicate (Na₂Si₂O₅).

Suitable polycarboxylates containing one carboxy group include lactic acid, glycolic acid and ether derivatives thereof as disclosed in Belgian Patent Nos. 831,368, 821,369 and 821,370. Polycarboxylates containing two carboxy groups include the water-soluble salts of succinic acid, malonic acid, (ethylenedioxy) diacetic acid, maleic acid, diglycollic acid, tartaric acid, tartronic acid and fumaric acid, as well as the ether carboxylates described in German Offenlegenschrift 2,446,686, and 2,446,687 and U.S. Patent No. 3,935,257 and the sulfinyl carboxylates described in Belgian Patent No. 840,623. Polycarboxylates containing three carboxy groups include, in particular, water-soluble citrates, aconitrates and citraconates as well as succinate derivatives such as the carboxymethyloxysuccinates described in British Patent No. 1,379,241, lactoxysuccinates described in Netherlands Application 7205873, and the oxypolycarboxylate materials such as 2-oxa-1,1,3-propane tricarboxylates described in British Patent No. 1,387,447.
Polycarboxylates containing four carboxy groups include oxydisuccinates disclosed in British Patent No. 1,261,829, 1,1,2,2-ethane tetracarboxylates, 1,1,3,3-propane tetracarboxylates and 1,1,2,3-propane tetracarboxylates. Polycarboxylates containing sulfo substituents include the sulfosuccinate derivatives disclosed in British Patent Nos. 1,398,421 and 1,398,422 and in U.S. Patent No. 3,936,448, and the sulfonated pyrolysed citrates described in British Patent No. 1,082,179, while polycarboxylates containing phosphone substituents are disclosed in British Patent No. 1,439,000.

Alicyclic and heterocyclic polycarboxylates include cyclopentane-cis,cis,cis-tetracarboxylates, cyclopentadienide pentacarboxylates, 2,3,4,5-tetrahydro-furan - cis, cis, cis-tetracarboxylates, 2,5-tetrahydro-furan -cis - dicarboxylates, 2,2,5,5-tetrahydrofuran - tetracarboxylates, 1,2,3,4,5,6-hexane -hexa-carboxylates and and carboxymethyl derivatives of polyhydric alcohols such as sorbitol, mannitol and xylitol. Aromatic poly-carboxylates include mellitic acid, pyromellitic acid and the phthalic acid derivatives disclosed in British Patent No. 1,425,343. Of the above, the preferred polycarboxylates are hydroxycarboxylates containing up to three carboxy groups per molecule, more particularly citrates.

Preferred builder systems for use in the present compositions include a mixture of a water-insoluble aluminosilicate builder such as zeolite A or of a layered silicate (SKS-6), and a water-soluble carboxylate chelating agent such as citric acid. More preferred builders are zeolite A and sodium tripolyphosphate.
Preferred builder systems for use in liquid detergent compositions of the present invention are soaps and polycarboxylates.

Other builder materials that can form part of the builder system for use in granular compositions include inorganic materials such as alkali metal carbonates, bicarbonates, silicates, and organic materials such as the organic phosphonates, amino polyalkylene phosphonates and amino polycarboxylates.

Other suitable water-soluble organic salts are the homo- or co-polymeric acids or their salts, in which the polycarboxylic acid comprises at least two carboxyl radicals separated from each other by not more than two carbon atoms. Polymers of this type are disclosed in GB-A-1,596,756. Examples of such salts are polyacrylates of MW 2000-5000 and their copolymers with maleic anhydride, such copolymers having a molecular weight of from 20,000 to 70,000, especially about 40,000.

Detergency builder salts are normally included in the compositions of the present invention at a level of from 5% to 80%, preferably from 10% to 70%, more preferably from 30% to 60% by weight of the composition.

### Chelating Agents

The laundry detergent and/or fabric care compositions herein may also optionally contain one or more iron and/or manganese chelating agents. Such chelating agents can be selected from the group consisting of amino carboxylates, amino phosphonates, polyfunctionally-substituted aromatic chelating agents and mixtures therein, all as hereinafter defined. Without intending to be bound by theory, it is believed that the benefit of these materials is due in part to their exceptional ability to remove iron and manganese ions from washing solutions by formation of soluble chelates.

Amino carboxylates useful as optional chelating agents include ethylenediaminetetracetates, N-hydroxyethylethylenediaminetriacetates, nitrilotriacetates, ethylenediamine tetraproprionates, triethylenetetraaminehexacetates, diethylenetriaminepentaacetates, and ethanoldiglycines, alkali metal, ammonium, and substituted ammonium salts therein and mixtures therein.

Amino phosphonates are also suitable for use as chelating agents in the compositions of the present invention when at least low levels of total phosphorus are permitted in detergent compositions, and include ethylenediaminetetrakis (methylenephosphonates) as DEQUEST. Preferred, these amino phosphonates do not contain alkyl or alkenyl groups with more than about 6 carbon atoms.

Polyfunctionally-substituted aromatic chelating agents are also useful in the compositions herein. See U.S. Patent 3,812,044, issued May 21, 1974, to Connor et al. Preferred compounds of this type in acid form are dihydroxydisulfobenzenes such as 1,2-dihydroxy-3,5-disulfobenzene.

A preferred biodegradable chelator for use herein is ethylenediamine disuccinate ("EDDS"), especially the [S,S] isomer as described in U.S. Patent 4,704,233, November 3, 1987, to Hartman and Perkins.

The compositions herein may also contain water-soluble methyl glycine diacetic acid (MGDA) salts (or acid form) as a chelant or co-builder useful with, for example, insoluble builders such as zeolites, layered silicates and the like.

If utilized in the compositions of the present invention, these chelating agents will generally be comprised at a level of from 0.1% to 15%, preferably from 0.1% to 3.0% by weight of the composition.

### Suds suppressor

Another optional ingredient for the laundry detergent and/or fabric care compositions of the present invention, are suds suppressor exemplified by silicones and silica-silicone mixtures. Silicones can be generally represented by alkylated polysiloxane materials while silica is normally used in finely divided forms exemplified by silica aerogels and xerogels and hydrophobic silicas of various types. These materials can be incorporated as particulates in which the suds suppressor is advantageously releasably incorporated in a water-soluble or water-dispersible, substantially non-surface-active detergent impermeable carrier. Alternatively the suds suppressor can be dissolved or dispersed in a liquid carrier and applied by spraying on to one or more of the other components.
A preferred silicone suds controlling agent is disclosed in Bartollota et al. U.S. Patent 3 933 672. Other particularly useful suds suppressors are the self-emulsifying silicone suds suppressors, described in German Patent Application DTOS 2 646 126 published April 28, 1977. An example of such a compound is DC-544, commercially available from Dow Corning, which is a siloxane-glycol copolymer. Especially preferred suds controlling agent are the suds suppressor system comprising a mixture of silicone oils and 2-alkyl-alcanols. Suitable 2-alkyl-alkanols are 2-butyl-octanol which are commercially available under the trade name lsofol 12 R. Such suds suppressor system are described in Copending European Patent application N 92870174.7 filed 10 November, 1992.
Especially preferred silicone suds controlling agents are described in Copending European Patent application N°92201649.8. Said compositions can comprise a silicone/silica mixture in combination with fumed nonporous silica such as AerosilR.

The suds suppressors described above are normally employed in the compositions of the present invention, at levels of from 0.001% to 2%, preferably from 0.01 % to 1% by weight of the composition.

### Dispersants

Suitable dispersants for use in laundry detergent and/or fabric care compositions of the present invention are water-soluble organic salts such as homo- or co-polymeric acids or their salts, in which the polycarboxylic acid comprises at least two carboxyl radicals separated from each other by not more than two carbon atoms. Polymers of this type are disclosed in GB-A-1,596,756. Examples of such salts are polyacrylates of MW 2000-5000 and their copolymers with maleic anhydride, such copolymers having a molecular weight of from 1,000 to 100,000. Especially, copolymer of acrylate and methylacrylate such as the 480N having a molecular weight of 4000, at a level from 0.5-20% by weight of composition can be added in the laundry detergent and/or fabric care compositions of the present invention.

Other suitable dispersants can be lime soap peptiser compounds, which have preferably a lime soap dispersing power (LSDP), as defined hereinafter of no more than 8, preferably no more than 7, most preferably no more than 6. The lime soap peptiser compound is preferably present at a level from 0.0001 % to 20% by weight.
A numerical measure of the effectiveness of a lime soap peptiser is given by the lime soap dispersant power (LSDP) which is determined using the time soap dispersant test as described in an article by H.C. Borghetty and C.A. Bergman, J. Am. Oil. Chem. Soc., volume 27, pages 88-90, (1950). This lime soap dispersion test method is widely used by practitioners in this art field being referred to, for example, in the following review articles; W.N. Linfield, Surfactant science Series, Volume 7, page 3; W.N. Linfield, Tenside surf. det., volume 27, pages 159-163, (1990); and M.K. Nagarajan, W.F. Masler, Cosmetics and Toiletries, volume 104, pages 71-73, (1989). The LSDP is the % weight ratio of dispersing agent to sodium oleate required to disperse the lime soap deposits formed by 0.025g of sodium oleate in 30ml of water of 333ppm CaCo₃ (Ca:Mg=3:2) equivalent hardness.
Polymeric lime soap peptisers suitable for use herein are described in the article by M.K. Nagarajan, W.F. Masler, to be found in Cosmetics and Toiletries, volume 104, pages 71-73, (1989).
Hydrophobic bleaches such as 4-[N-octanoyl-6-aminohexanoyl]benzene sulfonate, 4-[N-nonanoyl-6-aminohexanoyl]benzene sulfonate, 4-[N-decanoyl-6-aminohexanoyl]benzene sulfonate and mixtures thereof; and nonanoyloxy benzene sulfonate together with hydrophilic / hydrophobic bleach formulations can also be used as lime soap peptisers compounds.
Surfactants may also be used as lime soap peptiser compounds, such as certain amine oxides, betaines, sulfobetaines, alkyl ethoxysulfates and ethoxylated alcohols. Exemplary surfactants having a LSDP of no more than 8 for use in accord with the present invention include C₁₆-C₁₈ dimethyl amine oxide, C₁₂₋C₁₈ alkyl ethoxysulfates with an average degree of ethoxylation of from 1-5, particularly C₁₂-C₁₅ alkyl ethoxysulfate surfactant with a degree of ethoxylation of amount 3 (LSDP=4), and the C₁₄-C₁₅ ethoxylated alcohols with an average degree of ethoxylation of either 12 (LSDP=6) or 30, sold under the tradenames Lutensol A012 and Lutensol A030 respectively, by BASF GmbH.

### Dye transfer inhibitors (DTI)

The laundry detergent and/or fabric care compositions of the present invention can also include compounds for inhibiting dye transfer from one fabric to another of solubilized and suspended dyes encountered during fabric laundering operations involving colored fabrics. These DTI compounds are generally comprised at a level of from 0.001% to 10 %, preferably from 0.01% to 2%, more preferably from 0.05% to 1% by weight of the total composition. The DTI polymers have the ability to complex or adsorb the fugitive dyes washed out of dyed fabrics before the dyes have the opportunity to become attached to other articles in the wash. Especially suitable polymeric dye transfer inhibiting agents are polyamine N-oxide polymers, copolymers of N-vinylpyrrolidone and N-vinylimidazole, polyvinylpyrrolidone polymers, polyvinyloxazolidones and polyvinylimidazoles or mixtures thereof. Addition of such polymers also enhances the performance of the enzymes according the invention.

### a) Polyamine N-oxide polymers :

The polyamine N-oxide polymers suitable for use contain units having the following structure formula : 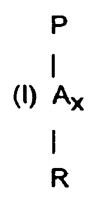 wherein P is a polymerisable unit, whereto the R-N-O group can be attached to or wherein the R-N-O group forms part of the polymerisable unit or a combination of both.
A is 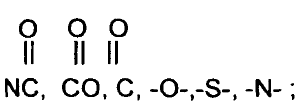 x is O or 1;
R are aliphatic, ethoxylated aliphatics, aromatic, heterocyclic or alicyclic groups or any combination thereof whereto the nitrogen of the N-O group can be attached or wherein the nitrogen of the N-O group is part of these groups.

The N-O group can be represented by the following general structures : 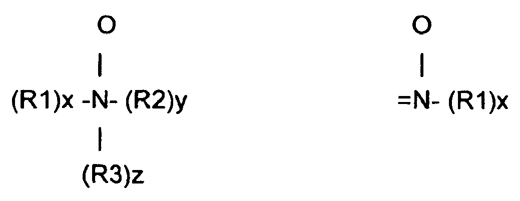 wherein R1, R2, and R3 are aliphatic groups, aromatic, heterocyclic or alicyclic groups or combinations thereof, x or/and y or/and z is 0 or 1 and wherein the nitrogen of the N-O group can be attached or wherein the nitrogen of the N-O group forms part of these groups.

The N-O group can be part of the polymerisable unit (P) or can be attached to the polymeric backbone or a combination of both.
Suitable polyamine N-oxides wherein the N-O group forms part of the polymerisable unit, comprise polyamine N-oxides wherein R is selected from aliphatic, aromatic, alicyclic or heterocyclic groups.
One class of said polyamine N-oxides comprises the group of polyamine N-oxides wherein the nitrogen of the N-O group forms part of the R-group. Preferred polyamine N-oxides are those wherein R is a heterocyclic group such as pyrridine, pyrrole, imidazole, pyrrolidine, piperidine, quinoline, acridine and derivatives thereof.
Another class of said polyamine N-oxides comprises the group of polyamine N-oxides wherein the nitrogen of the N-O group is attached to the R-group.
Other suitable polyamine N-oxides are the polyamine oxides whereto the N-O group is attached to the polymerisable unit.

Preferred class of these polyamine N-oxides are the polyamine N-oxides having the general formula (I) wherein R is an aromatic, heterocyclic or alicyclic groups wherein the nitrogen of the N-0 functional group is part of said R group.
Examples of these classes are polyamine oxides wherein R is a heterocyclic compound such as pyrridine, pyrrole, imidazole and derivatives thereof. Another preferred class of polyamine N-oxides are the polyamine oxides having the general formula (I) wherein R are aromatic, heterocyclic or alicyclic groups wherein the nitrogen of the N-0 functional group is attached to said R groups.
Examples of these classes are polyamine oxides wherein R groups can be aromatic such as phenyl.
Any polymer backbone can be used as long as the amine oxide polymer formed is water-soluble and has dye transfer inhibiting properties. Examples of suitable polymeric backbones are polyvinyls, polyalkylenes, polyesters, polyethers, polyamide, polyimides, polyacrylates and mixtures thereof.
The amine N-oxide polymers of the present invention typically have a ratio of amine to the amine N-oxide of 10:1 to 1:1000000. However the amount of amine oxide groups present in the polyamine oxide polymer can be varied by appropriate copolymerization or by appropriate degree of N-oxidation. Preferably, the ratio of amine to amine N-oxide is from 2:3 to 1:1000000. More preferably from 1:4 to 1:1000000, most preferably from 1:7 to 1:1000000. The polymers of the present invention actually encompass random or block copolymers where one monomer type is an amine N-oxide and the other monomer type is either an amine N-oxide or not. The amine oxide unit of the polyamine N-oxides has a PKa < 10, preferably PKa < 7, more preferred PKa < 6.
The polyamine oxides can be obtained in almost any degree of polymerisation. The degree of polymerisation is not critical provided the material has the desired water-solubility and dye-suspending power.
Typically, the average molecular weight is within the range of 500 to 1000,000; preferably from 1,000 to 50,000, more preferably from 2,000 to 30,000, most preferably from 3,000 to 20,000.

### b) Copolymers of N-vinylpyrrolidone and N-vinylimidazole :

The N-vinylimidazole N-vinylpyrrolidone polymers suitable for use in the compositions of the present invention, have an average molecular weight range from 5,000-1,000,000, preferably from 5,000-200,000. Highly preferred polymers comprise a polymer selected from N-vinylimidazole N-vinylpyrrolidone copolymers wherein said polymer has an average molecular weight range from 5,000 to 50,000 more preferably from 8,000 to 30,000, most preferably from 10,000 to 20,000.
The average molecular weight range was determined by light scattering as described in Barth H.G. and Mays J.W. Chemical Analysis Vol 113,"Modern Methods of Polymer Characterization".
Highly preferred N-vinylimidazole N-vinylpyrrolidone copolymers have an average molecular weight range from 5,000 to 50,000; more preferably from 8,000 to 30,000; most preferably from 10,000 to 20,000.
The N-vinylimidazole N-vinylpyrrolidone copolymers characterized by having said average molecular weight range provide excellent dye transfer inhibiting properties while not adversely affecting the cleaning performance of laudnry detergent and/or fabric care compositions formulated therewith.
The N-vinylimidazole N-vinylpyrrolidone copolymer of the present invention has a molar ratio of N-vinylimidazole to N-vinylpyrrolidone from 1 to 0.2, more preferably from 0.8 to 0.3, most preferably from 0.6 to 0.4 .

### c) Polyvinylpyrrolidone :

Suitable polyvinylpyrrolidones ("PVP") for use in the present invetnion, have an average molecular weight of from 2,500 to 400,000, preferably from 5,000 to 200,000, more preferably from 5,000 to 50,000, and most preferably from 5,000 to 15,000.
Suitable polyvinylpyrrolidones are commercially vailable from ISP Corporation, New York, NY and Montreal, Canada under the product names PVP K-15 (viscosity molecular weight of 10,000), PVP K-30 (average molecular weight of 40,000), PVP K-60 (average molecular weight of 160,000), and PVP K-90 (average molecular weight of 360,000). Other suitable polyvinylpyrrolidones which are commercially available from BASF Cooperation include Sokalan HP 165 and Sokalan HP 12; polyvinylpyrrolidones known to persons skilled in the detergent field (see for example EP-A-262,897 and EP-A-256,696).

### d) Polyvinyloxazolidone :

Suitable polyvinyloxazolidones for use in the present invention, have an average molecular weight of from 2,500 to 400,000, preferably from 5,000 to 200,000, more preferably from 5,000 to 50,000, and most preferably from 5,000 to 15,000.

### e) Polyvinylimidazole :

Suitable polyvinylimidazoles for use in the present invention, have an average of 2,500 to 400,000, preferably from 5,000 to 200,000, more preferably from 5,000 to 50,000, and most preferably from 5,000 to 15,000.

### f) Cross-linked polymers :

Suitable for use in the compositions of the present invention are cross-linked polymers. Cross-linked polymers are polymers whose backbone- are interconnected to a certain degree; these links can be of chemical or physical nature, possibly with active groups n the backbone or on branches; cross-linked polymers have been described in the Journal of Polymer Science, volume 22, pages 1035-1039..
In one embodiment, the cross-linked polymers are made in such a way that they form a three-dimensional rigid structure, which can entrap dyes in the pores formed by the three-dimensional structure. In another embodiment, the cross-linked polymers entrap the dyes by swelling.
Such cross-linked polymers are described in the co-pending patent application 94870213.9

### Colour care and fabric care benefits

Other suitable technologies which provide a type of colour care benefit can also be included. Examples of these technologies are metallo catalysts for colour maintenance. Such metallo catalysts are described in copending European Patent Application EP 596 184. Dye fixing agents, polyolefin dispersion for anti-wrinkles and improved water absorbancy, perfume and amino-functional polymer for colour care treatment and perfume substantivity are further examples of colour care / fabric care technologies and are described in the co-pending Patent Application EP 841 390, filed November 07, 1996.

Other suitable fabric softening may be inorganic or organic in type. Inorganic softening agents are exemplified by the smectite clays disclosed in GB-A-1 400 898 and in USP 5,019,292. Organic fabric softening agents include the water insoluble tertiary amines as disclosed in GB-A1 514 276 and EP-BO 011 340 and their combination with mono C12-C14 quaternary ammonium salts are disclosed in EP-B-0 026 527 and EP-B-0 026 528 and di-long-chain amides as disclosed in EP-B-0 242 919. Other useful organic ingredients of fabric softening systems include high molecular weight polyethylene oxide materials as disclosed in EP-A-0 299 575 and 0 313 146. Levels of smectite clay are normally in the range from 2% to 20%, more preferably from 5% to 15% by weight, with the material being added as a dry mixed component to the remainder of the formulation.

Organic fabric softening agents such as the water-insoluble tertiary amines or dilong chain amide materials are incorporated at levels of from 0.5% to 5% by weight, normally from 1% to 3% by weight whilst the high molecular weight polyethylene oxide materials and the water soluble cationic materials are added at levels of from 0.1% to 2%, normally from 0.15% to 1.5% by weight. These materials are normally added to the spray dried portion of the composition, although in some instances it may be more convenient to add them as a dry mixed particulate, or spray them as molten liquid on to other solid components of the composition.

### Bleaching agent

Other detergent ingredients that can be included in the laundry detergent and/or fabric care compositions of the present invention include bleaching agents.

The bleaching agent component for use herein can be any of the bleaching agents useful for cleaning compositions including oxygen bleaches as well as others known in the art. The bleaching agent suitable for the present invention can be an activated or non-activated bleaching agent.

One category of oxygen bleaching agent that can be used encompasses percarboxylic acid bleaching agents and salts thereof. Suitable examples of this class of agents include magnesium monoperoxyphthalate hexahydrate, the magnesium salt of meta-chloro perbenzoic acid, 4-nonylamino-4-oxoperoxybutyric acid and diperoxydodecanedioic acid. Such bleaching agents are disclosed in U.S. Patent 4,483,781, U.S. Patent Application 740,446, European Patent Application 0,133,354 and U.S. Patent 4,412,934. Highly preferred bleaching agents also include 6-nonylamino-6-oxoperoxycaproic acid as described in U.S. Patent 4,634,551.

Another category of bleaching agents that can be used encompasses the halogen bleaching agents. Examples of hypohalite bleaching agents, for example, include trichloro isocyanuric acid and the sodium and potassium dichloroisocyanurates and N-chloro and N-bromo alkane sulphonamides. Such materials are normally added at 0.5-10% by weight of the finished product, preferably 1-5% by weight.

Also suitable activators are acylated citrate esters such as disclosed in Copending European Patent Application No. 91870207.7.

Useful bleaching agents, including peroxyacids and bleaching systems comprising bleach activators and peroxygen bleaching compounds for use in the compositions of the present invention are described in our co-pending applications WO 95/10592, WO 97/00937, WO95/27772, WO95/27773, WO95/27774 and WO95/27775.

The hydrogen peroxide may also be present by adding an enzymatic system (i.e., an enzyme and a substrate therefore) which is capable of generating hydrogen peroxide at the beginning or during the washing and/or rinsing process. Such enzymatic systems are disclosed in EP 537 381 filed October 9, 1991.

### Others

Other components such as soil-suspending agents, soil-release agents, optical brighteners, abrasives, bactericides, tarnish inhibitors, and/or coloring agents may be employed.

Antiredeposition and soil suspension agents suitable herein include cellulose derivatives such as methylcellulose, carboxymethylcellulose and hydroxyethylcellulose, and homo- or co-polymeric polycarboxylic acids or their salts. Polymers of this type include the polyacrylates and maleic anhydride-acrylic acid copolymers previously mentioned as builders, as well as copolymers of maleic anhydride with ethylene, methylvinyl ether or methacrylic acid, the maleic anhydride constituting at least 20 mole percent of the copolymer. These materials are normally used at levels of from 0.5% to 10%, preferably from 0.75% to 8%, more preferably from 1% to 6% by weight of the composition.

Other useful polymeric materials are the polyethylene glycols, particularly those of molecular weight 1000-10000, more particularly 2000 to 8000 and most preferably 4000. These are used at levels of from 0.20% to 5% more preferably from 0.25% to 2.5% by weight. These polymers and the previously mentioned homo- or co-polymeric polycarboxylate salts are valuable for improving whiteness maintenance, fabric ash deposition, and cleaning performance on clay, proteinaceous and oxidizable soils in the presence of transition metal impurities.

It is well known in the art that free chlorine in tap water rapidly deactivates the enzymes comprised in detergent compositions. Therefore, using chlorine scavenger such as perborate, ammonium sulfate, sodium sulphite or polyethylene imine at a level above 0.1% by weight of total composition, in the formulas will provide improved through the wash stability of the detergent enzymes. Compositions comprising chlorine scavenger are described in the European patent application EP 553 607 filed January 31, 1992.

Alkoxylated polycarboxylates such as those prepared from polyacrylates are useful herein to provide additional grease removal performance. Such materials are described in WO 91/08281. Chemically, these materials comprise polyacrylates having one ethoxy side-chain per every 7-8 acrylate units. The side-chains are of the formula -(CH₂CH₂O)ₘ(CH₂)ₙCH₃ wherein m is 2-3 and n is 6-12. The side-chains are ester-linked to the polyacrylate "backbone" to provide a "comb" polymer type structure. The molecular weight can vary, but is typically in the range of 2000 to 50,000. Such alkoxylated polycarboxylates can comprise from 0.05% to 10%, by weight, of the compositions herein.

### Method of washing

The compositions of the invention may be used in essentially any washing, cleaning and/or fabric care methods, including soaking methods, pre-treatment methods, methods with rinsing steps for which a separate rinse aid composition may be added and post-treatment methods.

The process described herein comprises contacting fabrics with a laundering solution in the usual manner and exemplified hereunder. A conventional laundry method comprises treating soiled fabric with an aqueous liquid having dissolved or dispensed therein an effective amount of the laundry detergent and/or fabric care composition. The method of cleaning is preferably carried out at 5°C to 95°C, especially between 10°C and 60°C. The pH of the treatment solution is preferably from 7 to 12.

The following examples are meant to exemplify compositions of the present invention, but are not necessarily meant to limit or otherwise define the scope of the invention.

In the compositions, the enzymes levels are expressed by pure enzyme by weight of the total composition and unless otherwise specified, the detergent ingredients are expressed by weight of the total compositions. The abbreviated component identifications therein have the following meanings:
- LAS :: Sodium linear C₁₁₋₁₃ alkyl benzene sulphonate.
- TAS :: Sodium tallow alkyl sulphate.
- CxyAS :: Sodium C₁ₓ - C_{1y} alkyl sulfate.
- CxyEz :: C₁ₓ - C_{1y} predominantly linear primary alcohol condensed with an average of z moles of ethylene oxide.
- CxyEzS :: C₁ₓ - C_{1y} sodium alkyl sulfate condensed with an average of z moles of ethylene oxide.
- QAS 1 :: R₂N+(CH₃)₂(C₂H₄OH) with R₂ = C₈-C₁₁.
- CFAA :: C₁₂-C₁₄ alkyl N-methyl glucamide.
- TPKFA :: C₁₂-C₁₄ topped whole cut fatty acids.
- Silicate :: Amorphous Sodium Silicate (SiO₂:Na₂O ratio = 1.6-3.2).

- Zeolite A :: Hydrated Sodium Aluminosilicate of formula Na₁₂(A1O₂SiO₂)₁₂. 27H₂O having a primary particle size in the range from 0.1 to 10 micrometers (Weight expressed on an anhydrous basis).
- Na-SKS-6 :: Crystalline layered silicate of formula δ-Na₂Si₂O_{5.}
- Citric :: Anhydrous citric acid.
- Carbonate :: Anhydrous sodium carbonate with a particle size between 200 and 900 micrometres.
- Sulphate :: Anhydrous sodium sulphate.
- Mg Sulphate :: Anhydrous magnesium sulfate.
- STPP :: Sodium tripolyphosphate.
- TSPP :: Tetrasodium pyrophosphate.
- MA/AA :: Random copolymer of 4:1 acrylate/maleate, average molecular weight about 70,000-80,000.
- PB1 :: Anhydrous sodium perborate monohydrate of nominal formula NaBO₂.H₂O₂.
- PB4 :: Sodium perborate tetrahydrate of nominal formula NaBO₂.3H₂O.H₂O₂.
- Percarbonate :: Anhydrous sodium percarbonate of nominal formula 2Na₂CO₃.3H₂O₂.
- TAED :: Tetraacetylethylenediamine.
- CBD-NACA-OBS :: Reaction product of Cellulose Binding Domain from the cellulase sold under the tradename Carezyme by Novo Nordisk A/S with polytyrosine and (6-nonamidocaproyl) oxybenzene sulfonate molecules.
- DTPA :: Diethylene triamine pentaacetic acid.
- HEDP :: 1,1-hydroxyethane diphosphonic acid.
- DETPMP :: Diethyltriamine penta (methylene) phosphonate, marketed by Monsanto under the Trade name Dequest 2060.
- EDDS :: Ethylenediamine-N,N'-disuccinic acid, (S,S) isomer in the form of its sodium salt
- Photoactivated Bleach :: Sulfonated zinc phtalocyanine encapsulated in dextrin soluble polymer.
- Protease :: Proteolytic enzyme sold under the tradename Savinase, Alcalase by Novo Nordisk A/S, the "protease D" variant with the substitution set N76D/S103A/V1041 and the protease described in PCT application Nos. PCT/US98/22588, PCT/US98/22482 and PCT/US98/22486 with the amino acid substitution set 101 G/103A/1041/159D/232V/236H/245R/248D/252K.
- Amylase :: Amylolytic enzyme sold under the tradename Termamyl ® and Duramyl® available from Novo Nordisk A/S and those variants having improved thermal stability with amino acid deletions R181^{*} + G182^{*} or T183^{*} + G184^{*} as described in WO95/35382.
- Lipase :: Lipolytic enzyme sold under the tradename Lipolase, Lipolase Ultra by Novo Nordisk A/S and Lipomax by Gist-Brocades.
- Cellulase :: Cellulytic enzyme sold under the tradename Carezyme, Celluzyme and/or Endolase by Novo Nordisk A/S.
- CMC :: Sodium carboxymethyl cellulose.
- PVP :: Polyvinyl polymer, with an average molecular weight of 60,000.
- PVNO :: Polyvinylpyridine-N-Oxide, with an average molecular weight of 50,000.
- PVPVI :: Copolymer of vinylimidazole and vinylpyrrolidone, with an average molecular weight of 20,000.
- Brightener 1 :: Disodium 4,4'-bis(2-sulphostyryl)biphenyl.
- Brightener 2 :: Disodium 4,4'-bis(4-anilino-6-morpholino-1.3.5-triazin-2-yl) stilbene-2:2'-disulfonate.
- Silicone antifoam :: Polydimethylsiloxane foam controller with siloxane-oxyalkylene copolymer as dispersing agent with a ratio of said foam controller to said dispersing agent of 10:1 to 100:1.
- Suds Suppressor :: 12% Silicone/silica, 18% stearyl alcohol,70% starch in granular form.
- SRP 1 :: Anionically end capped poly esters.
- HMWPEO :: High molecular weight polyethylene oxide.
- PEO :: Polyethylene oxide, with an average molecular weight of 5,000.
- CBD-Rotundial :: Reaction product of CBD Cellulozome from *Clostridium cellulovorans*, which is sold under the tradename Cellulose Binding Domain by Sigma with Rotundial molecules.
- CBD-Damascone :: Reaction product of CBD Cellulozome from *Clostridium cellulovorans,* which is sold under the tradename Cellulose Binding Domain by Sigma with polylysine and α-Damascone molecules.

### Example 1

According to the present invention, this example illustrates the coupling of a perfume citral, hygiene agent glutaraldehyde or insect control agent citronellal to a CBD, as a deposition aid.

The reaction at equal mol takes place in mild conditions (pH 6 to 9.5, from 1 to 48hours / See for example Wirth, P at al 1991 Biorg.Chem 19, 133, 1991 and Chamow SM et al Bioconjugate chem, 4, 133 1994). The chemicals such as perfume (citral), insect control agent (citronellal) and hygiene agent (glutaraldehyde) are linked to NH2 groups of the CBD, linking region and/or polyreactive linking region via Schiffs base reaction. The reaction could also be completed in anhydrous ethanol that contain a drying agent such as Sodium Sulphate.

In case the CBD possesses more than one Lysine or there is a polyreactive linking region comprising more than one lysine, the reaction is identical with the appropriate number of chemicals per CBD. For example 10 equivalent of aldehyde will be added per mol of CBD from familly III that contain more than 10 Lysines.

For example, the CBD having at least one Lysine, has a NH₂ reactive moiety to be linked to perfume. The CBD is non volatile and does not change the character of the aldehyde.

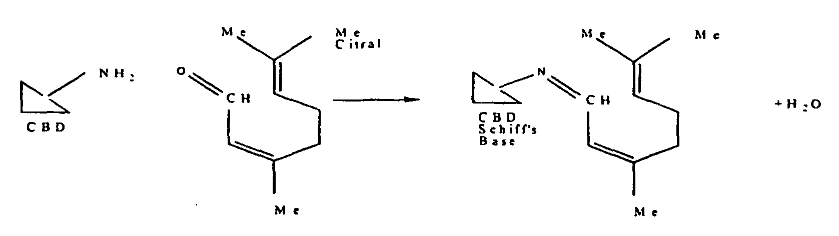

Improved release on dry fabrics is obtained by the slow hydrolysis of the CBD perfume linkage.

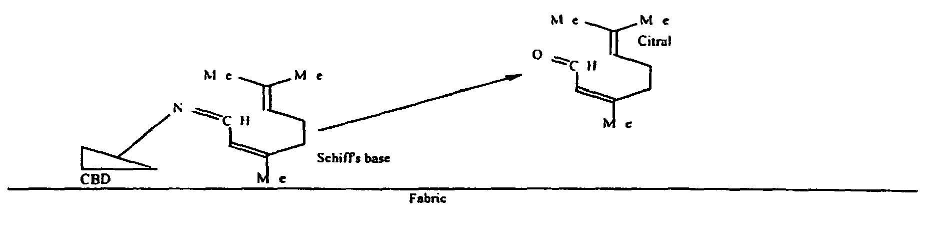

### Example 2

The following high density laundry detergent compositions were prepared according to the present invention :

| | I | II |
|---|---|---|
| LAS | 6.0 | 6.0 |
| TAS | - | 0.1 |
| C25AS | 4.0 | 3.0 |
| C28AS | 1.0 | 2.0 |
| C25E5 | 4.6 | 4.0 |
| C25E3S | 5.0 | 4.5 |
| QAS 1 | 0.5 | 1.0 |
| Zeolite A | 20.0 | 20.0 |
| Citric | - | 2.5 |
| Carbonate | 10.0 | 13.0 |
| Na-SKS-6 | 10.0 | 10.0 |
| Silicate | 0.5 | 0.3 |
| Sulfate | - | 14 |
| Mg sulfate | - | 0.2 |
| MA/AA | 1.0 | 1.0 |
| CMC | 0.4 | 0.4 |
| Percarbonate | 18.0 | - |
| TAED | 3.9 | - |
| SRP 1 | - | 0.2 |
| EDDS | 0.5 | 0.5 |
| HEDP | 0.4 | 0.4 |
| Protease | 0.05 | 0.03 |
| Amylase | 0.008 | 0.008 |
| Cellulase | 0.0007 | 0.001 |
| Lipase | 0.01 | 0.01 |
| Photoactivated bleach (ppm) | 20 | 20 |
| PVNO/PVPVI | - | 0.1 |
| Brightener 1 | 0.09 | 0.09 |
| Perfume | 0.4 | 0.4 |
| Silicone antifoam | 0.3 | 0.3 |
| CBD-Damascone | 5.0 | 1.0 |
| Density in g/litre | 850 | 850 |
| Miscellaneous and minors | Up to 100% | |

### Example 3

The following granular laundry detergent compositions of particular utility under European machine wash conditions were prepared according to the present invention :

| | I | II |
|---|---|---|
| LAS | 7.0 | 6.0 |
| TAS | 1.0 | 1.0 |
| C24AS/C25AS | 1.0 | 1.0 |
| C25E3S | 0.1 | 0.1 |
| C25E5 | 4.0 | 4.0 |
| STPP | - | 20.0 |
| Zeolite A | 17.0 | - |
| Na-SKS-6 | 5.0 | 5.0 |
| Carbonate | 15.0 | 10.0 |
| Sulfate | 12.0 | 2.0 |
| MA/AA | 1.0 | 1.0 |
| CMC | 0.4 | 0.4 |
| PB4 | 15.0 | 15.0 |
| TAED | 2.5 | 2.5 |
| CBD-NACA-OBS | 2.0 | 2.0 |
| DETPMP | 0.2 | 0.2 |
| HEDP | 0.3 | 0.3 |
| Protease | 0.02 | 0.02 |
| Lipase | 0.004 | 0.004 |
| Cellulase | 0.0007 | 0.0007 |
| Amylase | 0.003 | 0.003 |
| PVP | 0.9 | 0.9 |
| Photoactivated bleach (ppm) | 20 | 20 |
| Brightener 1 | 0.15 | 0.15 |
| Clay | - | 10 |
| HMWPFO | - | 0.2 |
| Perfume | 0.3 | 0.3 |
| Silicone antifoam | 2.0 | 2.0 |
| Density in g/litre | 650 | 650 |
| Miscellaneous and minors | Up to 100% | |

### Example 4

The following liquid detergent formulations were prepared according to the present invention (Levels are given in parts per weight) :

| | I | II |
|---|---|---|
| HLAS | 11.5 | 7.0 |
| C45E2.25S | 4.0 | 4.0 |
| C23E7 | 4.0 | 3.0 |
| CFAA | 4.0 | 2.0 |
| TPKFA | 10.0 | 6.0 |
| Citric (50%) | 5.0 | 3.0 |
| CaCl₂ | 0.01 | 0.01 |
| Boric acid | 2.0 | 1.0 |
| Na hydroxide | 0.7 | 1.5 |
| Ethanol | 1.75 | 2.0 |
| 1,2 Propanediol | 9.0 | 8.0 |
| Monoethanolamine | 8.0 | 3.0 |
| Protease | 0.03 | 0.02 |
| Lipase | 0.002 | 0.001 |
| Amylase | 0.002 | 0.002 |
| Cellulase | 0.001 | 0.0005 |
| SRP 1 | 0.2 | 0.1 |
| DTPA | 0.4 | 0.2 |
| PVNO | 0.4 | 0.2 |
| Brightener 1 | 0.2 | 0.1 |
| Silicone antifoam | 0.04 | 0.1 |
| CBD-Damascone | 2.0 | 1.0 |
| Miscellaneous and water | Up to 100% | |

### Example 5

The following laundry bar detergent compositions were prepared according to the present invention :

| | I |
|---|---|
| LAS | 15.0 |
| C28AS | - |
| Na Laurate | - |
| C45E7 | 2.0 |
| Zeolite A | - |
| Carbonate | 13.0 |
| Ca Carbonate | 30.0 |
| Sulfate | 3.0 |
| TSPP | - |
| STPP | 10.0 |
| Bentonite clay | - |
| DETPMP | 0.6 |
| CMC | 1.0 |
| Talc | 8.0 |
| Silicate | 4.0 |
| PVNO | - |
| MA/AA | - |
| SRP 1 | 0.3 |
| Amylase | 0.01 |
| Protease | 0.003 |
| Lipase | - |
| Cellulase | .0002 |
| PEO | - |
| Perfume | 0.3 |
| Mg sulfate | 3.0 |
| CBD-Damascone | 1.0 |
| CBD-Rotundial | 5.0 |
| Brightener 2 | 0.15 |
| Photoactivated bleach (ppm) | 15.0 |

### Example 6

The following granular fabric detergent compositions which provide "softening through the wash" capability were prepared according to the present invention :

| | I | II |
|---|---|---|
| C45AS | - | 10.0 |
| LAS | 7.6 | - |
| C68AS | 1.3 | - |
| C45E7 | 4.0 | - |
| C25E3 | - | 5.0 |
| Coco-alkyl-dimethyl hydroxy- | 1.4 | 1.0 |
| ethyl ammonium chloride | | |
| Citrate | 5.0 | 3.0 |
| Na-SKS-6 | - | 11.0 |
| Zeolite A | 15.0 | 15.0 |
| MA/AA | 4.0 | 4.0 |
| DETPMP | 0.4 | 0.4 |
| PB1 | 15.0 | - |
| Percarbonate | - | 15.0 |
| TAED | 5.0 | 5.0 |
| Smectite clay | 10.0 | 10.0 |
| HMWPEO | - | 0.1 |
| CBD-Damascone | 0.5 | 2.0 |
| Protease | 0.02 | 0.01 |
| Lipase | 0.02 | 0.01 |
| Amylase | 0.03 | 0.005 |
| Cellulase | 0.001 | - |
| Silicate | 3.0 | 5.0 |
| Carbonate | 10.0 | 10.0 |
| Suds suppressor | 1.0 | 4.0 |
| CMC | 0.2 | 0.1 |
| Miscellaneous and minors | Up to 100% | |

## Claims

1. A laundry detergent and/or fabric care composition comprising a chemical entity comprising or amino acid sequence comprising a cellulose binding domain and a benefit agent; **characterised in that** the composition further comprises a surfactant and a protease.

2. A composition according to claim 1 wherein said amino acid sequence comprising a cellulose binding domain is selected from CBDs CBHII from *Trichoderma reesei,* CBDs CenC, CenA and Cex from *Cellulomonas fimi,* CBD CBHI from *Trichoderma reesei,* CBD Cellulozome from *Clostridium cellulovorans,* CBD E3 from *Thermomonospora fusca,* CBD-dimer from *Clostridium stecorarium* (NCIMB11754) XynA, CBD from . *Bacillus agaradherens* (NCIMB40482), CBD family 45 from *Humicola insolens,* and/or mixtures thereof; preferably selected from CBD CenC from *Cellulomonas fimi,* CBD Cellulozome from *Clostridium cellulovorans,* CBD family 45 from *Humicola Insolens,* and/or mixtures thereof.

3. A composition according to claims 1-2 wherein said benefit agent is selected from perfumes, hygiene agents, insect control agents, fabric softening agents, soil release agents, bleaching agents, dye fixatives agents, brigtheners, latex, resins, and/or mixtures thereof, preferably from perfumes, hygiene agents, insect control agents, and/or mixtures thereof.

4. A composition according to claims 1-3 wherein said benefit agent is linked to said deposition aid via a linking region.

5. A composition according to claim 4 wherein said linking region is a amino acid linking region.

6. A composition according to claim 4 wherein said linking region is a polymer selected from PEG(NPC)2, (NH2)2-PEG, t-BOC-NH-PEG-NH2, MAL-PEG-NHS and/or VS-PEG-NHS polymers.

7. A composition according to any of the preceding claims wherein said benefit agent is linked to said deposition aid or to said linking region via a weak bond.

8. A composition according to any of the preceding claims wherein said chemical entity is comprised at a level of from 0.00001% to 50%, preferably from 0.001 % to 20%, more preferably from 0.1% to 10% by weight of the total composition.

9. A composition according to any of the preceding claims wherein said surfactant is selected from nonionic and anionic surfactants, preferably at a weight ratio of nonionic to anionic of at least 1:10.

10. A composition according to claim 9 wherein said nonionic surfactants are selected from polyethylene oxide condensates of alkyl phenols, polyhydroxy fatty acid amide and/or mixtures thereof.

11. A composition according to claim 9 wherein said anionic surfactants are selected from linear alkyl benzene sulfonate, alkyl ethoxylates sulfates, alkyl sulfates, mid-chain-branched anionics and/or mixtures thereof.

12. A composition according to any of the preceding claims wherein the surfactant system further comprises a cationic surfactant, preferably a detergent cationic surfactant.

13. A composition according to claim 12 wherein said detergent cationic surfactant is selected from coconut trimethyl ammonium chloride or bromide; coconut methyl dihydroxyethyl ammonium chloride or bromide; decyl dimethyl hydroxyethyl ammonium chloride or bromide; and/or mixtures thereof.

14. A composition according to claims 12-13 wherein the cationic surfactant is comprised at a level of no more than 5%, preferably from 1% to 2% by weight of the composition.

15. A composition according to any of the preceding claims wherein said protease is selected from : Protease B, Protease C, a variant of protease D having the amino acid substitution set 76/103/104 according to the numbering of *Bacillus lentus subtilisin,* a multiply-substituted protease variant comprising the amino acid substitution set 101/103/104/159/232/236/245/248/252 according to the numbering of *Bacillus amyloliquiefaciens subtilisin,* and/or mixtures thereof.

16. A composition according to any of the preceding claims further comprising an amylase.

17. A method of treating a fabric with a composition comprising a chemical entity, surfactant and a protease according to claims 1-16, for providing sanitation and/or insect control.

18. A method of treating a fabric with a composition comprising a chemical entity, surfactant and a protease according to claims 1-16, for providing fabric cleaning including soil and stain removal, dingy fabric cleaning and/or whiteness maintenance.

19. A method of treating a fabric with a composition comprising a chemical entity, surfactant and a protease according to claims 1-16 for providing fabric care including anti-wrinkling, anti-bobbling and anti-shrinkage properties and/or fabric softness

## Revendications

1. Composition détergente pour le lavage du linge et/ou pour le soin des tissus comprenant une entité chimique comprenant une séquence aminoacide comprenant un domaine de liaison à la cellulose et un agent bénéfique; **caractérisée en ce que** la composition comprend en outre un agent tensioactif et une protéase.

2. Composition selon la revendication 1, dans laquelle ladite séquence aminoacide comprenant un domaine de liaison à la cellulose est choisie parmi CBDs CBHII de *Trichoderma reesei,* CBDs CenC, CenA et Cex de *Cellulomonas fimi,* CBD CBHI de *Trichoderma reesei,* CBD Cellulozome de *Clostridium cellulovorans,* CBD E3 de *Thermomonospora fusca,* CBD-dimère de *Clostridium stecorarium* (NCIMB11754) XynA, CBD de *Bacillus agaradherens* (NCIMB40482), CBD famille 45 de *Humicola insolens,* et/ou leurs mélanges; choisie de préférence parmi CBD CenC de *Cellulomonas fimi,* CBD Cellulozome de *Clostridium cellulovorans,* CBD famille 45 de *Humicola Insolens,* et/ou leurs mélanges.

3. Composition selon la revendication 1 à 2, dans laquelle ledit agent bénéfique est choisi parmi les parfums, agents hygiéniques, agents insecticides, agents d'adoucissement des tissus, agents de libération des salissures, agents de blanchiment, agents fixateurs pour colorants, azurants, latex, résines, et/ou leurs mélanges, de préférence parmi les parfums, agents hygiéniques, agents insecticides, et/ou leurs mélanges.

4. Composition selon la revendication 1 à 3, dans laquelle ledit agent bénéfique est lié audit adjuvant de dépôt via une région de liaison.

5. Composition selon la revendication 4, dans laquelle ladite région de liaison est une région de liaison d'acide aminé.

6. Composition selon la revendication 4, dans laquelle ladite région de liaison est un polymère choisi parmi les polymères PEG(NPC)2, (NH2)2-PEG, t-BOC-NH-PEG-NH2, MAL-PEG-NHS et/ou VS-PEG-NHS.

7. Composition selon l'une quelconque des revendications précédentes, dans laquelle ledit agent bénéfique est lié audit adjuvant de dépôt ou à ladite région de liaison via une liaison faible.

8. Composition selon l'une quelconque des revendications précédentes, dans laquelle ladite entité chimique est comprise à un taux allant de 0,00001 % à 50 %, de préférence de 0,001 % à 20 %, plus préférablement de 0,1 % à 10 % en poids de la composition totale.

9. Composition selon l'une quelconque des revendications précédentes, dans laquelle ledit agent tensioactif est choisi parmi les agents tensioactifs non ioniques ou anioniques, de préférence à un rapport pondéral de non ionique sur anionique d'au moins 1:10.

10. Composition selon la revendication 9, dans laquelle lesdits agents tensioactifs non ioniques sont choisis parmi les condensats d'oxyde de polyéthylène d'alkylphénols, un amide d'acide gras polyhydroxyle et/ou leurs mélanges.

11. Composition selon la revendication 9, dans laquelle lesdits agents tensioactifs anioniques sont choisis parmi le benzène sulfonate d'alkyle linéaire, les sulfates d'éthoxylates d'alkyle, les sulfates d'alkyle, les anioniques ramifiés à mi-chaîne et/ou leurs mélanges.

12. Composition selon l'une quelconque des revendications précédentes, dans laquelle le système tensioactif comprend en outre un agent tensioactif cationique, de préférence un agent tensioactif cationique détergent.

13. Composition selon la revendication 12, dans laquelle ledit agent tensioactif cationique détergent est choisi parmi le chlorure ou bromure de noix de coco triméthylammonium; le chlorure ou bromure de noix de coco méthyl dihydroxyéthyle; le chlorure ou bromure de décyl diméthyl hydroxyéthyl ammonium; et/ou leurs mélanges.

14. Composition selon les revendications 12 à 13, dans laquelle l'agent tensioactif cationique est compris à un niveau de pas plus de 5 %, de préférence de 1 % à 2 % en poids de la composition.

15. Composition selon l'une quelconque des revendications précédentes, dans laquelle ladite protéase est choisie parmi: Protéase B, Protéase C, un variant de protéase D ayant l'ensemble de substitution d'acide aminé 76/103/104 selon la numérotation de *Bacillus lentus subtilisine,* un variant de protéase à substitution multiple comprenant l'ensemble de substitution d'acide aminé 101/103/104/159/232/236/245/248/252 selon la numérotation de *Bacillus amyloliquiefaciens subtilisine,* et/ou leurs mélanges.

16. Composition selon l'une quelconque des revendications précédentes, comprenant en outre une amylase.

17. Procédé de traitement d'un tissu avec une composition comprenant une entité chimique, un agent tensioactif et une protéase selon les revendications 1 à 16, pour fournir un assainissement et/ou un contrôle des insectes.

18. Procédé de traitement d'un tissu avec une composition comprenant une entité chimique, un agent tensioactif et une protéase selon les revendications 1 à 16, pour fournir un nettoyage de tissu incluant l'élimination de salissures et de taches, le nettoyage d'un tissu terne et/ou le maintien de la blancheur.

19. Procédé de traitement d'un tissu avec une composition comprenant une entité chimique, un agent tensioactif et une protéase selon les revendications 1 à 16, pour fournir un soin des tissus incluant des propriétés d'anti-froissage, d'anti-boulochage et d'anti-rétrécissement et/ou une douceur du tissu

## Patentansprüche

1. Wäschewaschmittel und/oder Textilpflegezusammensetzung, die eine chemische Einheit umfasst, die eine Aminosäure-Sequenz umfasst, die eine Cellulose bindende Domäne und einen Wirkstoff umfasst; **dadurch gekennzeichnet, dass** die Zusammensetzung ferner ein Tensid und eine Protease umfasst.

2. Zusammensetzung nach Anspruch 1, wobei die Aminosäure-Sequenz, die eine Cellulose bindende Domäne umfasst, ausgewählt ist aus CBDs CBHII von *Trichoderma reesei,* CBDs CenC, CenA und Cex von *Cellulomonas fimi,* CBD CBHI von *Trichoderma reesei,* CBD-Cellulosom von *Clostridium cellulovorans,* CBD E3 von *Thermomonospora fusca,* CBD-Dimer von *Clostridium stecorarium* (NCIMB 11754) XynA, CBD von *Bacillus agaradherens* (NCIMB40482), CBD-Familie 45 von *Humicola insolens* und/oder Mischungen davon; vorzugsweise ausgewählt aus CBD CenC von *Cellulomonas fimi,* CBD-Cellulosom von *Clostridium cellulovorans,* CBD-Familie 45 von *Humicola Insolens* und/oder Mischungen davon.

3. Zusammensetzung nach Ansprüchen 1-2, wobei der Wirkstoff aus Duftstoffen, Hygienemitteln, Insektenbekämpfungsmitteln, Textilweichmachern, Schmutzabweisemitteln, Bleichmitteln, Farbstofffixierungsmitteln, Aufhellern, Latex, Harzen und/oder Mischungen davon, vorzugsweise aus Duftstoffen, Hygienemitteln, Insektenbekämpfungsmitteln und/oder Mischungen davon ausgewählt ist.

4. Zusammensetzung nach Ansprüchen 1-3, wobei der Wirkstoff über einen Bindungsbereich mit dem Ablagerungshilfsmittel verbunden ist.

5. Zusammensetzung nach Anspruch 4, wobei der Bindungsbereich ein Aminosäure-Bindungsbereich ist.

6. Zusammensetzung nach Anspruch 4, wobei der Bindungsbereich ein Polymer ist, ausgewählt aus PEG(NPC)2-, (NH2)2-PEG-, t-BOC-NH-PEG-NH2-, MAL-PEG-NHS- und/oder VS-PEG-NHS-Polymeren.

7. Zusammensetzung nach einem der vorstehenden Ansprüche, wobei der Wirkstoff über eine schwache Bindung mit dem Ablagerungshilfsmittel oder dem Bindungsbereich verbunden ist.

8. Zusammensetzung nach einem der vorstehenden Ansprüche, wobei die chemische Einheit in einem Anteil von 0,00001 Gew.-% bis 50 Gew.-%, vorzugsweise von 0,001 Gew.-% bis 20 Gew.-%, mehr bevorzugt von 0,1 Gew.-% bis 10 Gew.-% der Gesamtzusammensetzung enthalten ist.

9. Zusammensetzung nach einem der vorstehenden Ansprüche, wobei das Tensid aus nichtionischen und anionischen Tensiden ausgewählt ist, vorzugsweise mit einem Gewichtsverhältnis von nichtionisch zu anionisch von mindestens 1:10.

10. Zusammensetzung nach Anspruch 9, wobei die nichtionischen Tenside aus Polyethylenoxidkondensaten von Alkylphenolen, Polyhydroxyfettsäureamid und/oder Mischungen davon ausgewählt sind.

11. Zusammensetzung nach Anspruch 9, wobei die anionischen Tenside aus linearem Alkylbenzolsulfonat, Alkylethoxylatsulfaten, Alkylsulfaten, mittelkettig verzweigten anionischen Verbindungen und/oder Mischungen davon ausgewählt sind.

12. Zusammensetzung nach einem der vorstehenden Ansprüche, wobei das Tensidsystem ferner ein kationisches Tensid umfasst, vorzugsweise ein kationisches Reinigungstensid.

13. Zusammensetzung nach Anspruch 12, wobei das kationische Reinigungstensid aus Kokosnusstrimethylammoniumchlorid oder -bromid; Kokosnussmethyldihydroxyethylammoniumchlorid oder -bromid; Decyldimethylhydroxyethylammoniumchlorid oder -bromid und/oder Mischungen davon ausgewählt ist.

14. Zusammensetzung nach Ansprüchen 12-13, wobei das kationische Tensid zu einem Anteil von nicht mehr als 5 Gew.-%, vorzugsweise von 1 Gew.-% bis 2 Gew.-% der Zusammensetzung enthalten ist.

15. Zusammensetzung nach einem der vorstehenden Ansprüche, wobei die Protease ausgewählt ist aus: Protease B, Protease C, einer Variante von Protease D mit dem Aminosäure-Substitutionsset 76/103/104 nach der Nummerierung von *Bacillus lentus subtilisin,* einer mehrfach substituierten Proteasevariante, die das Aminosäure-Substitutionsset 101/103/104/159/232/236/245/248/252 nach der Nummerierung von *Bacillus amyloliquiefaciens subtilisin* umfasst, und/oder Mischungen davon.

16. Zusammensetzung nach einem der vorstehenden Ansprüche, die weiterhin eine Amylase umfasst.

17. Verfahren zur Behandlung eines Stoffes mit einer Zusammensetzung, die eine chemische Einheit, Tensid und eine Protease nach Ansprüchen 1-16 umfasst, für die Bereitstellung von Desinfektion und/oder Insektenbekämpfung.

18. Verfahren zur Behandlung eines Stoffes mit einer Zusammensetzung, die eine chemische Einheit, Tensid und eine Protease nach Ansprüchen 1-16 umfasst, für die Bereitstellung von Textilreinigung, einschließlich Schmutz- und Fleckenentfennung, Reinigung schmutziger Textilien und/oder Weißbeständigkeit.

19. Verfahren zur Behandlung eines Stoffes mit einer Zusammensetzung, die eine chemische Einheit, Tensid und eine Protease nach Ansprüchen 1-16 umfasst, für die Bereitstellung von Textilpflege, einschließlich Antiknitter-, Antiknötchenbildungs- und Antieinlaufeigenschaften und/oder Textilweichheit.
